# FASCICULE DE BREVET EUROPEEN

(11) **EP 1 150 731 B1**
(45) Date de publication et mention de la délivrance du brevet: **02.11.2006**
(21) Numéro de dépôt: 00903785.4
(22) Date de dépôt: 10.02.2000
(51) Int. Cl.: A61M 16/00

(54) **DISPOSITIF D'ALIMENTATION EN GAZ POUR APNEES DU SOMMEIL**
VORRICHTUNG ZUR GASZUFUHR BEI SCHLAFAPNOE
GAS SUPPLY DEVICE FOR SLEEP APNEA

(30) Priorité: 12.02.1999 FR 9901738
(43) Date de publication de la demande: 07.11.2001
(73) Titulaire: Mallinckrodt Developpement France, 54600 Villers-les-Nancy (FR)
(72) Inventeur: NADJAFIZADEH, Hossein, F-54600 Villers-les-Nancy (FR); NICOLAZZI, Pascal, F-54520 Laxou (FR); GRILLER LANOIR, Véronique, F-25000 Besançon (FR)
(74) Mandataire: Geismar, Thierry
(86) Numéro de dépôt international: PCT/FR2000/000331
(87) Numéro de publication internationale: WO 2000/047260

(56) Documents cités:
- WO-A-92/11054
- WO-A-94/06499
- WO-A-94/23780
- WO-A-97/14462
- WO-A-97/28838

## Description

L'invention concerne un procédé de commande d'un appareil de fourniture de pression d'air à un patient souffrant de troubles du sommeil.

L'invention concerne également un appareil de fourniture de pression d'air à un patient souffrant de troubles du sommeil.

Ces troubles du sommeil sont respiratoires et tendent à réveiller intempestivement le patient.

Ce sont par exemple les apnées, les hypopnées, les vibrations acoustiques ou ronflements, la limitation du flux respiratoire dues à un resserrement des voies aériennes supérieures du patient.

Le document US-A-5 458 137 décrit un procédé et un dispositif pour contrôler la respiration en cas de troubles du sommeil, qui utilisent des niveaux de pression multiples et variables. WO 97/ 288 38 divulgue les caractéristiques du préambule de la revendication 1.

Une source de pression fournit un gaz respirable comprimé à une pression relativement basse aux voies aériennes de l'utilisateur.

Des capteurs de pression surveillent les pressions et les convertissent en signaux électriques.

Les signaux électriques sont filtrés et traités pour extraire des caractéristiques spécifiques telles que la durée et des niveaux d'énergie.

Si ces caractéristiques dépassent des seuils choisis de durée et de niveau d'énergie au-delà d'une période minimum de temps, le microprocesseur indique la présence d'un trouble respiratoire du sommeil.

Si un nombre choisi de ces événements apparaît pendant une période de temps choisie, le microprocesseur ajuste la pression fournie par la source.

Le document US-A-5 490 502 décrit un procédé et un appareil pour optimiser la pression positive commandée afin de minimiser le débit d'air provenant d'un générateur tout en assurant que la limitation de débit dans les voies aériennes du patient ne se produit pas.

Il y est prévu de détecter la limitation de débit en analysant une onde de débit respiratoire.

Dès que la présence d'une limitation de débit a été analysée, le système détermine une action à effectuer pour l'ajustement de la pression positive commandée.

La pression est augmentée, diminuée ou maintenue selon que la limitation de débit a été détectée et en fonction des actions précédentes mises en oeuvre par le système.

Citons également les documents US-A-5 335 654, EP-A-661 071 et EP-A-651 971.

L'invention vise à améliorer les procédés et dispositifs de l'état de la technique, à adapter automatiquement et continûment la pression délivrée à l'état du patient et à prévenir et empêcher l'apparition des troubles en prévoyant un appareil de fourniture de pression d'air faisant passer un indicateur d'apparition de troubles d'un état (ON) à un état (OFF).

A cet effet, l'invention concerne un appareil de fourniture de pression d'air à un patient souffrant de troubles du sommeil tels qu'apnée, selon la revendication 1.

Le patient porte un masque par lequel de l'air sous pression est fourni à ses voies aériennes supérieures par l'appareil.

Selon l'invention, il est prévu un algorithme de commande utilisant un signal de débit de sortie de l'appareil pour la détection d'apnée, d'hypopnée, d'événements de limitation de débit, de fuites, et utilsant l'analyse d'une information de pression pour déterminer la présence d'un ronflement, également appelé vibrations acoustiques.

La pression fournie aux voies aériennes supérieures du patient par l'appareil peut être maintenue constante, être augmentée ou diminuée en fonction de la détermination de l'événement qui a été effectuée par l'algorithme de commande.

Ainsi, si aucune respiration n'est détectée par l'algorithme de commande en un temps minimum prédéterminé dépendant d'un temps de respiration moyen calculé, on détermine la présence d'une apnée.

Ce temps minimum prédéterminé de détection d'apnée est par exemple égal à une constante de temps, par exemple 10 s, ajoutée à un facteur de proportionnalité multiplié par le temps de respiration moyen calculé, ce facteur étant par exemple égal à 5/8.

Pour chaque apnée, le signal de débit de sortie est amplifié et filtré pour déterminer la présence ou l'absence d'oscillations cardiaques.

Si des oscillations cardiaques ont été détectées pendant le dernier intervalle de temps écoulé, par exemple égal à 5 s, alors l'apnée est classée comme étant centrale et aucune commande ne se produit dans l'algorithme.

Si aucune oscillation cardiaque n'a été détectée dans cet intervalle de temps, l'apnée est classée comme étant obstructive, et la pression est augmentée d'une valeur prédéterminée une première fois et, durant la même apnée, deux autres fois régulièrement, par exemple toutes les 15 s.

L'algorithme de commande compare des variations de débit crête à crête durant la dernière respiration du patient par rapport à un nombre prédéterminé de respirations précédentes, par exemple égal à 8.

Après chaque respiration, on effectue une classification en :
- respiration normale, si la dernière valeur de débit crête à crête est comprise dans une fourchette déterminée par rapport à la valeur moyenne sur les 8 respirations précédentes, par exemple de 40 % à 150 % ou 140 % de celles-ci ;
- respiration hypopnéique, si la dernière valeur de débit est en-deçà de cette plage ;
- respiration hyperpnéique, si la dernière valeur de débit est au-delà de cette plage.

Une détermination d'hypopnée est effectuée si la détection de respiration hypopnéique s'est produite pendant au moins un temps déterminé, par exemple 10 s, et prend fin après un nombre déterminé de respirations normales ou hyperpnéiques, par exemple égal à 2.

Une détermination d'hypopnée conduit à une augmentation de pression déterminée, par exemple de 1 cm H2O d'abord, puis, durant la même hypopnée, à une augmentation de pression d'une autre valeur déterminée et ce régulièrement, par exemple de 0,5 cm H2O toutes les deux respirations hypopnéiques.

L'algorithme de commande analyse et compare, respiration par respiration, la forme d'onde du débit respiratoire avec une forme d'onde sinusoïdale de même période et de même pente.

Après la comparaison basée sur deux critères de forme de débit, chaque respiration est d'abord classée en tant que normale, intermédiaire ou à débit limité.

Une classification finale basée sur la combinaison de la classification de flux et de l'occurrence de ronflements, change le classement des respirations de normales en intermédiaires, respectivement d'intermédiaires en respiration à débit limité.

Il est décidé d'un traitement lorsqu'un certain nombre, par exemple 2, respirations successives à débit limité ou un certain nombre, par exemple 5, de respirations successives intermédiaires ont lieu après par exemple deux respirations normales.

Ce traitement provoque une augmentation de pression déterminée, répétée régulièrement un certain nombre de fois, par exemple de 0,3 cm H2O trois fois toutes les deux respirations.

Pour chaque respiration, le signal de pression est amplifié et filtré pour détecter la présence ou l'absence de vibrations acoustiques ou ronflement.

Une détermination d'un ronflement valide est effectuée par l'algorithme de commande, si la vibration acoustique détectée s'est produite au moins pendant un certain temps, par exemple 7 % de la durée moyenne des trois dernières respirations, et avec une période inférieure à un facteur proportionnel à ce temps moyen, par exemple 120 % de celui-ci.

Dans le cas d'un ronflement valide, l'algorithme augmente la pression d'une valeur déterminée, par exemple de 1 cm H2O, si la dernière commande due à un ronflement s'est produite depuis plus d'un temps déterminé, par exemple 1 minute.

On détermine une fuite moyenne comme étant égale au débit moyen durant la respiration.

L'algorithme de commande compare continûment la fuite actuelle à une limite de fuite, laquelle limite peut être réglée à partir de la pression.

Si la fuite actuelle dépasse la limite, on bloque toutes les commandes d'augmentation de pression générées à la suite de détections d'événements.

Après une détection d'une apnée ou d'un événement de ronflement ou une commande en hypopnée ou une décision de traitement, l'algorithme diminuera la pression d'une valeur déterminée, par exemple de 0,5 cm H2O, dans une première étape après un temps déterminé, par exemple 5 minutes, et régulièrement pour les diminutions suivantes, par exemple toutes les minutes.

Une pression de maintien déterminée, par exemple de 8 cm H2O est fournie par l'appareil si aucune respiration n'a été détectée pendant un temps déterminé, par exemple de deux minutes, ou si la pression fournie a été supérieure ou égale à une valeur déterminée pendant un temps déterminé, par exemple à 17 cm H2O pendant 10 ou 30 minutes.

Un avantage du procédé est une adaptation automatique des critères de détection aux caractéristiques respiratoires du patient.

Ainsi, toute modification du rythme respiratoire est prise en compte par l'algorithme pour effectuer la détection.

Le fait de faire intervenir une valeur moyenne de temps de cycle respiratoire sur un certain nombre de cycles respiratoires précédents a comme effet le suivi régulier des variations du cycle et d'amplitude respiratoire et une meilleure détection.

L'invention sera mieux comprise à la lecture de la description qui va suivre, faite en référence aux figures.
La figure 1 est un schéma montrant l'appareil de fourniture de pression d'air au patient.
La figure 2 représente un algorithme de prise de décision en vue d'une première commande d'augmentation de pression.
La figure 3 représente un algorithme d'indication d'apparitions de troubles.
La figure 4 représente un algorithme de qualification de respiration.

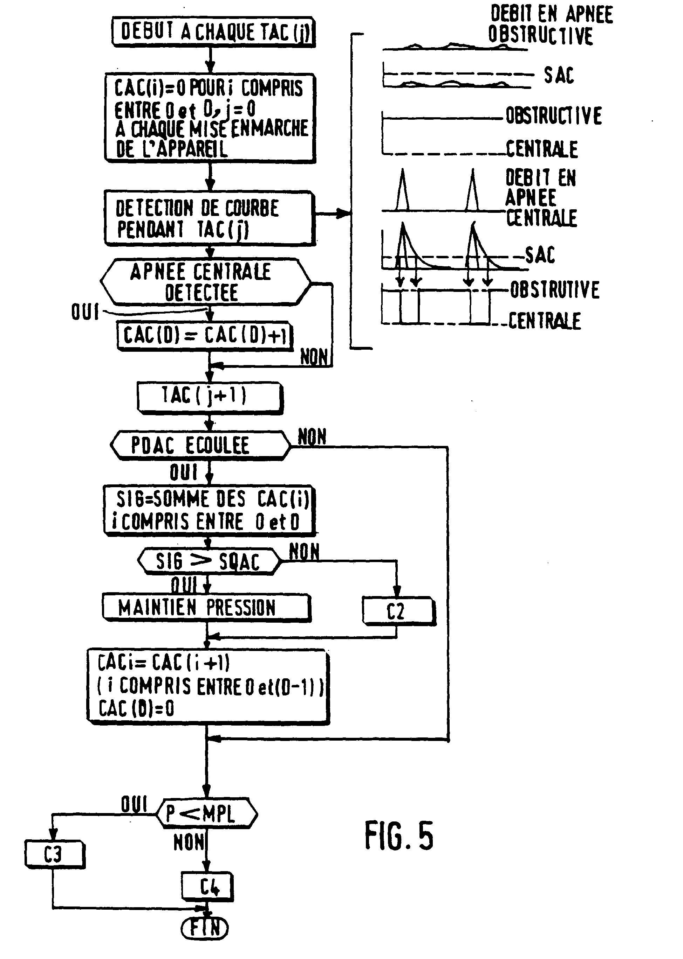

La figure 5 représente un algorithme de détection d'apnée centrale et obstructive et de commande de pression en fonction du résultat de ces détections, ainsi qu'un algorithme de diminution de pression selon l'apparition précédente ou non d'évènements représentatifs de troubles du sommeil.
La figure 6 représente un algorithme de qualification de cycle de ventilation normale, à hyperventilation ou à hypoventilation.
La figure 7 représente un algorithme de détection de respiration hypopnéique.
La figure 8 représente un algorithme de détection de respiration hyperpnéique.
La figure 9 représente un algorithme de détection de respiration normale.
La figure 10 représente un algorithme de détection de pression élevée.
La figure 11 représente un algorithme de détection de fuite du masque.
La figure 12 représente un algorithme de détection de vibrations acoustiques.

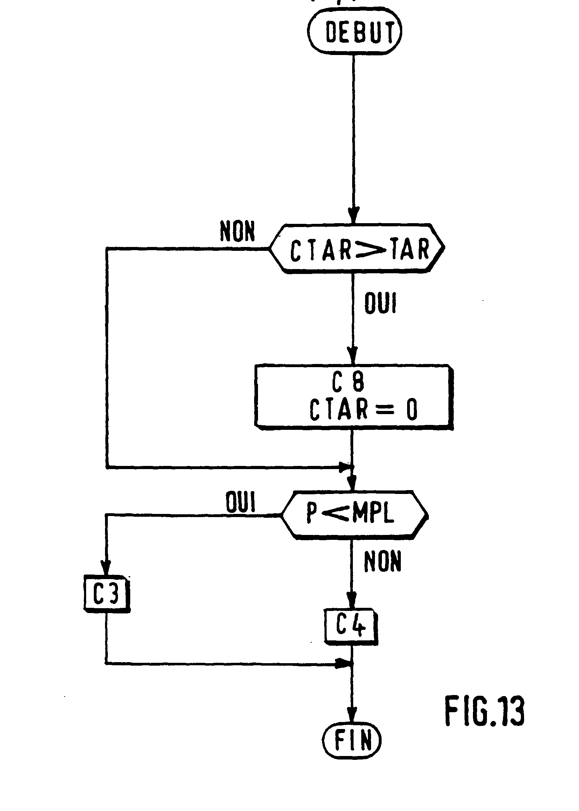

La figure 13 représente un algorithme de diminution de pression en cas de détection de vibrations acoustiques.

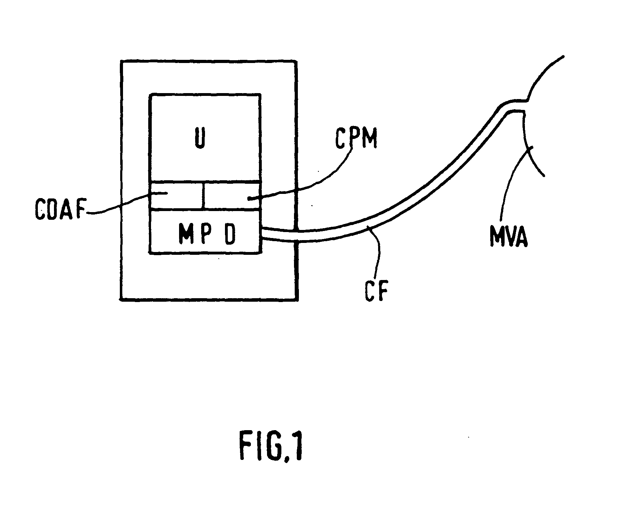

A la figure 1, l'appareil de fourniture de pression d'air à un patient comporte une unité U centrale de traitement et de commande de pression, un module MPD commandé de fourniture de pression, un masque MVA pour les voies aériennes supérieures du patient, un conduit CF de fourniture de pression d'air du module MPD au masque MVA.

On mesure le débit d'air fourni au patient et la pression d'air régnant dans le masque MVA, par un capteur CDAF de débit d'air fourni, relié à l'unité centrale U et par un capteur CPM de pression dans le masque MVA, relié à l'unité centrale U.

On détermine à partir des variables mesurées, si des événements représentatifs de troubles du sommeil apparaissent ou non.

Les algorithmes du procédé suivant l'invention sont mis en oeuvre par un logiciel intégré à l'unité centrale U.

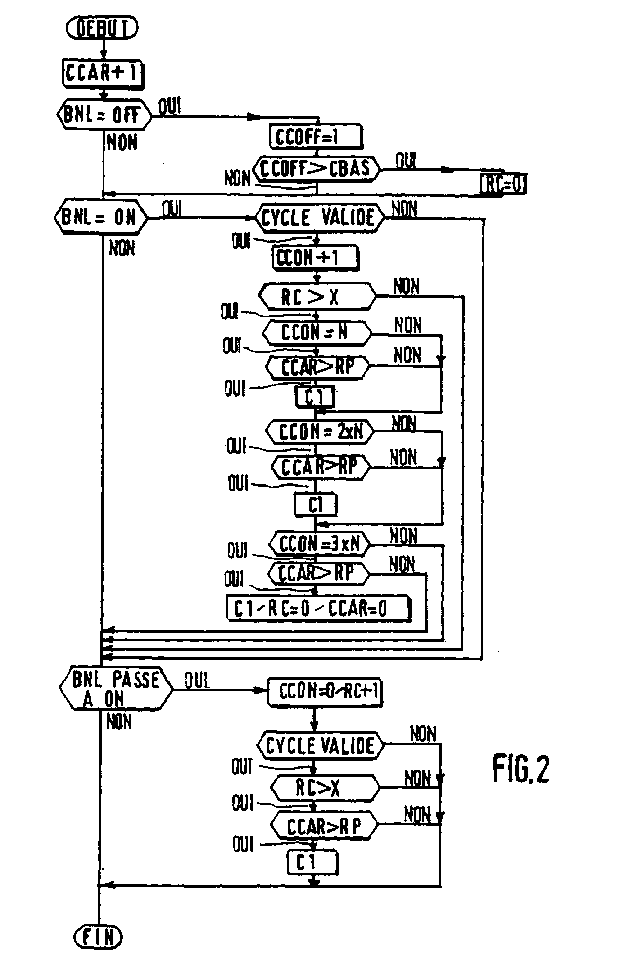

A la figure 2, on détermine à partir des variables mesurées si le cycle respiratoire actuel du patient correspond à un cycle respiratoire valide prédéterminé.

On met dans un premier état ON d'apparition de troubles un indicateur BLN d'apparition de troubles, si l'apparition de l'un ou plusieurs des évènements représentatifs de troubles du sommeil est déterminée.

On met l'indicateur BLN dans un deuxième état OFF d'absence de troubles, si l'apparition des événements représentatifs de troubles du sommeil n'est pas déterminée.

On compte un premier nombre CCAR de cycles respiratoires valides déterminés depuis la dernière commande de pression.

On compte un deuxième nombre CCON de cycles respiratoires valides déterminés depuis le dernier passage de l'indicateur BLN au premier état ON.

On compte un troisième nombre RC de passages successifs de l'indicateur BLN du deuxième état OFF au premier état ON.

Lorsque l'indicateur BLN se trouve dans le premier état ON, on commande par la commande C1 une première augmentation déterminée de pression d'air fourni, lorsque, à la fois :
- le cycle respiratoire actuel a été déterminé comme étant valide ;
- le premier nombre CCAR est supérieur à un premier nombre RP entier prédéterminé ;
- le deuxième nombre CCON correspond à un autre ou plusieurs deuxièmes nombres N entiers prédéterminés ;
- le troisième nombre RC est supérieur ou égal à un troisième nombre X entier prédéterminé.

Lorsque l'indicateur BLN passe du deuxième état OFF au premier état ON, on commande par la commande C1 la première augmentation déterminée de pression d'air fourni, lorsque, seulement et à la fois :
- le cycle respiratoire actuel a été déterminé comme étant valide ;
- le premier nombre CCAR est supérieur à un premier nombre RP entier prédéterminé ;
- le troisième nombre RC est supérieur ou égal à un troisième nombre X entier prédéterminé.

Dans une réalisation, les deuxièmes nombres entiers N sont compris entre 1 et 300.

Dans une autre réalisation, les deuxièmes nombres entiers N sont les trois premiers multiples d'un entier N₀ déterminé.

Dans une autre réalisation, les deuxièmes nombres entiers N sont respectivement 2, 4 et 6, N₀ étant égal à 2.

Dans une autre réalisation, le premier nombre entier RP prédéterminé est compris entre 1 et 255.

Dans une autre réalisation, le premier nombre entier RP prédéterminé est égal à 10.

Dans une autre réalisation, le troisième nombre entier X prédéterminé est compris entre 1 et 100.

Dans une autre réalisation, le troisième nombre entier X prédéterminé est égal à 1.

Dans une autre réalisation, la première commande C1 déterminée d'augmentation de pression est inférieure à + 10 mbar.

Dans une autre réalisation, la première commande C1 déterminée d'augmentation de pression est sensiblement égale à + 0,3 mbar.

On remet à 0 les premiers et troisièmes nombres CCAR ; RC de cycles respiratoires valides comptés et de passages comptés, après que le deuxième nombre CCON compté de cycles valides a atteint le plus grand des deuxièmes nombres N entiers prédéterminés.

On remet à 0 le deuxième nombre compté CCON lorsque l'indicateur BLN passe du deuxième état. OFF au premier état ON.

Le cycle respiratoire valide prédéterminé correspond à un maximum de débit respiratoire supérieur à une valeur de débit prédéterminée telle que 50 ml/s, à un volume inspiratoire supérieur à une valeur de volume prédéterminée telle que 0,05 I et à une absence de saturation lors de la détection de débit.

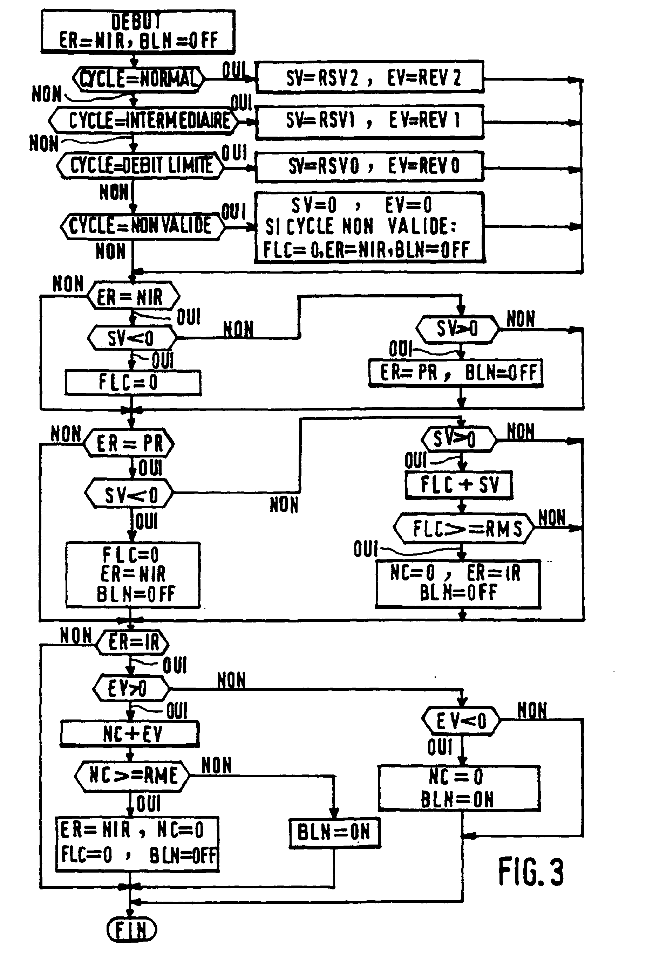

A la figure 3, pour donner l'état ON ou OFF à l'indicateur BLN d'apparition de troubles,
- on initialise, lors de la mise en marche de l'appareil, une variable ER d'état à un troisième état d'absence NIR de traitement et l'indicateur BLN au deuxième état OFF.

Puis, séquentiellement,
- on qualifie, à partir des variables mesurées, les cycles respiratoires comme appartenant à différentes catégories telles que cycle à débit limité, cycle intermédiaire, cycle normal et cycle non valide, correspondant chacune respectivement à des pondérations RSV0, REV0; RSV1, REV1 ; RSV2, REV2 ; 0, 0 ;
- on affecte les pondérations de la catégorie du cycle actuellement qualifié à des premier et deuxième accumulateurs SV ; EV de pondération ;
- si le cycle qualifié appartient à la catégorie de cycle non valide, on remet la variable d'état ER au troisième état NIR et l'indicateur BLN au deuxième état OFF et on initialise un premier compteur FLC à une valeur prédéterminée .

Si l'état de la variable d'état ER correspond au troisième état NIR :
- si la valeur d'un premier accumulateur SV est inférieure à une première valeur comparative, on réinitialise le compteur FLC à sa valeur prédéterminée ;
- si la valeur du premier accumulateur SV est sensiblement égale à sa première valeur comparative, on se dispense d'action et on passe au test suivant ;
- si la valeur du premier accumulateur SV est supérieure à sa première valeur comparative, on fait passer la variable d'état ER à un quatrième état PR de possibilité de traitement et on met l'indicateur BLN au deuxième état OFF.

Si l'état de la variable ER d'état correspond au quatrième état PR et
- si la valeur du premier accumulateur SV est inférieure à sa première valeur comparative, on réinitialise le premier compteur FLC à sa valeur prédéterminée, on remet la variable d'état ER et l'indicateur BLN respectivement aux troisième et deuxième états NIR ; OFF ;
- si la valeur du premier accumulateur SV est sensiblement égale à sa première valeur comparative, on se dispense d'action et on passe au test suivant ;
- si la valeur du premier accumulateur SV est supérieure à sa première valeur comparative, on fait prendre au premier compteur FLC sa valeur précédente additionnée de la valeur du premier accumulateur SV et si alors la valeur du premier compteur FLC est supérieure ou égale à une butée haute RMS prédéterminée :
   ■ on réinitialise un deuxième compteur NC à une valeur prédéterminée ;
   ■ on fait passer la variable d'état ER à un cinquième état IR de traitement ; et
   ■ on fait passer l'indicateur BLN au premier état ON.

Si l'état de la variable d'état ER correspond au cinquième état IR de traitement :
• si la valeur du deuxième accumulateur EV est supérieure à une deuxième valeur comparative, on fait prendre au deuxième compteur NC sa valeur précédente additionnée de la valeur du deuxième accumulateur EV et
   ■ si alors la valeur du deuxième compteur NC est supérieure ou égale à une butée basse RME, on remet la variable d'état ER et l'indicateur BLN respectivement aux troisièmes et deuxièmes états NIR ; OFF et on réinitialise les premier et deuxième compteurs FLC ; NC à leurs valeurs respectives prédéterminées;
   ■ ou sinon, on fait passer l'indicateur BLN à son premier état ON ;
• si la valeur du deuxième accumulateur EV est inférieure à sa deuxième valeur comparative, on réinitialise le deuxième compteur NC à sa valeur respective prédéterminée et on fait passer l'indicateur BLN au premier état ON
• si la valeur du deuxième accumulateur EV est sensiblement égale à sa deuxième valeur comparative, on se dispense d'action.

Dans une réalisation, les pondérations RSV2, REV2; RSV1, REV1 ; RSV0, REV0 ; 0 ,0 correspondant aux catégories de cycle normal, cycle intermédiaire, cycle à débit limité et cycle non valide, sont respectivement sensiblement égales à -1 ; 1 ; 5 et 0 pour le premier accumulateur SV et sont respectivement sensiblement égales à 1 ; -1 ; -1 et 0 pour le deuxième accumulateur EV.

Les première et deuxième valeurs comparatives et les valeurs prédéterminées d'initialisation des premier et deuxième compteurs FLC ; NC sont chacune sensiblement égales à 0.

Les butées haute et basse RMS ; RME sont respectivement sensiblement égales à 10 et 2.

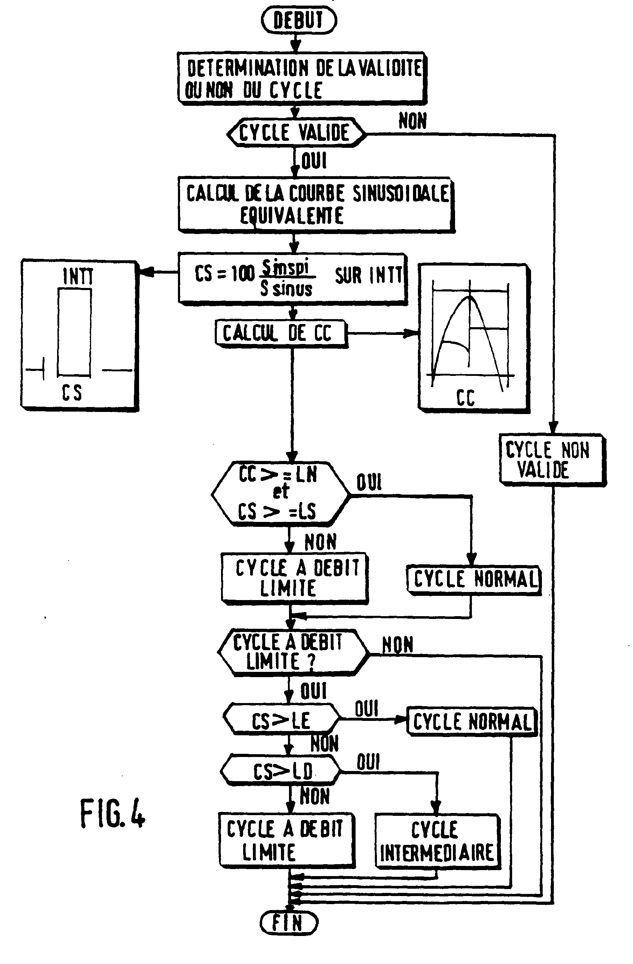

A la figure 4, on qualifie les cycles respiratoires mesurés.

Le cycle respiratoire valide prédéterminé correspond à un maximum de débit inspiratoire supérieur à une valeur de débit prédéterminée telle que 50 ml/s, à un volume inspiratoire supérieur à une valeur de volume prédéterminée telle que 0,05 l, à une absence de saturation lors de la détection de débit, à un temps inspiratoire mesuré compris dans un intervalle prédéterminé tel que de 0,5 s à 6 s et à une durée mesurée de cycles respiratoires comprise dans un autre intervalle prédéterminé tel que de 1,5 s à 20 s.

Si le cycle respiratoire mesuré est déterminé comme étant valide, alors
- on calcule une courbe sinusoïdale équivalente respectant des caractéristiques prédéterminées par rapport à la courbe inspiratoire du cycle inspiratoire mesuré ;
- on calcule un critère de surface CS proportionnel au rapport de l'aire délimitée par la courbe inspiratoire sur l'aire délimitée par la courbe sinusoïdale équivalente, chacune étant prise sur un même intervalle de temps, compris dans la phase inspiratoire du cycle respiratoire mesuré ;
- on calcule un critère de corrélation CC entre la courbe inspiratoire du cycle inspiratoire mesuré et la courbe sinusoïdale équivalente ;
- si le critère CC de corrélation calculé est supérieur ou égal à une première limite normale prédéterminée LN, et si le critère de surface calculé CS est supérieur à une deuxième limite prédéterminée LS de surface, on qualifie le cycle respiratoire mesuré de normal et sinon, on le qualifie de cycle à débit limité.

Si le cycle respiratoire mesuré a été qualifié de cycle à débit limité,
- si le critère de surface CS calculé est supérieur à une troisième limite prédéterminée LE d'expert, on requalifie le cycle respiratoire mesuré de normal,
- ou sinon,
   ■ si le critère de surface CS calculé est supérieur à une quatrième limite prédéterminée LD de débit, on requalifie le cycle respiratoire mesuré d'intermédiaire,
   ■ et dans le cas contraire, on le qualifie de cycle à débit limité.

La deuxième limite LS de surface, la quatrième limite LD de débit et la troisième limite LE d'expert étant prédéterminées dans un ordre croissant.

Les caractéristiques prédéterminées de la courbe sinusoïdale équivalente comprennent une demi-période sensiblement égale au temps inspiratoire mesuré et une pente à l'origine sensiblement égale à celle de la courbe inspiratoire lorsqu'elle atteint sensiblement un tiers de son amplitude maximale.

Dans une réalisation, le critère de surface calculé CS est sensiblement égal à cent fois le rapport des aires prises chacune de sensiblement un quart à trois quart de la durée de la phase inspiratoire du cycle respiratoire mesuré .

Le critère de corrélation calculé CC est sensiblement égal au maximum de cent fois les coefficients de corrélation entre la courbe inspiratoire et la courbe sinusoïdale équivalente prises respectivement sur la seconde moitié de la phase inspiratoire et sur la totalité de cette-ci.

Les première, deuxième, quatrième et troisième limites LN ; LS ; LD ; LE étant respectivement comprises entre 45 et 100 ; 0 et 100 ; 0 et 100 ; 0 et 100 et étant par exemple sensiblement égales à 87 ; 40 ; 60 et 90 respectivement.

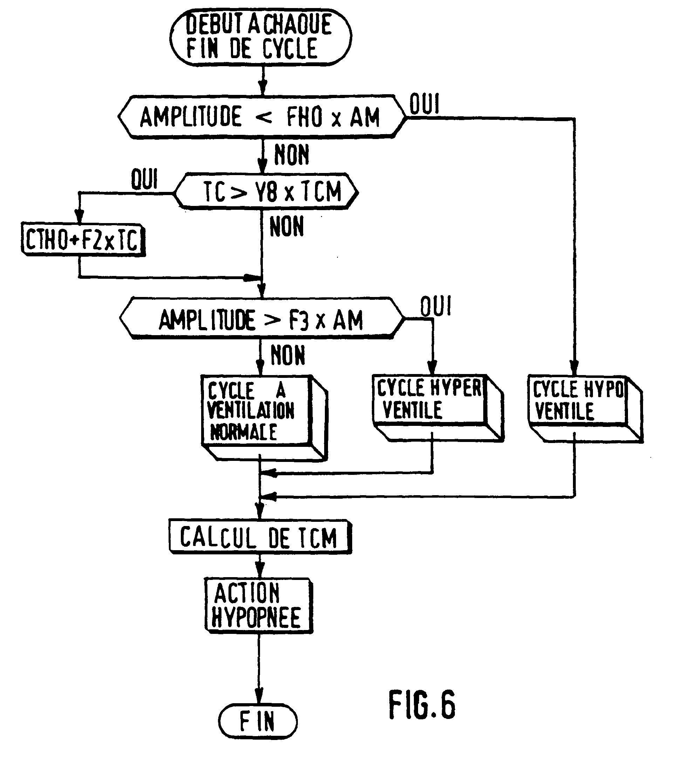

Aux figures 6 à 9, on qualifie les cycles respiratoires d'hyperventilés, d'hypoventilés ou de cycles à ventilation normale et on génère des commandes de presssion en fonction des qualifications effectuées.

On calcule à chaque fin de cycle respiratoire mesuré, l'amplitude moyenne AM sur un quatrième nombre prédéterminé Y4 de cycles respiratoires précédents.

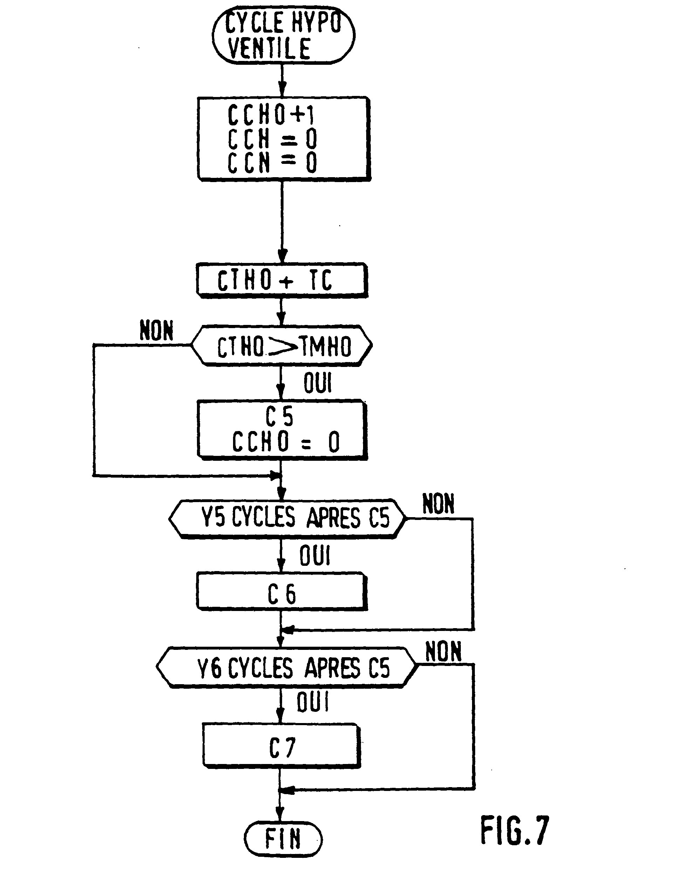

Comme représenté à la figure 7, si l'amplitude mesurée du dernier cycle respiratoire est inférieure à l'amplitude moyenne AM calculée multipliée par un premier facteur d'hypopnée prédéterminé FHO, alors on ajoute à un compteur CTHO de temps en hypopnée la durée TC du dernier cycle respiratoire mesuré,
- si la valeur actuelle du compteur de temps en hypopnée CTHO est supérieure ou égaie à un temps minimum d'hypopnée TMHO, on commande par une commande C5 une cinquième augmentation prédéterminée de pression,
- après la fin d'un cinquième nombre prédéterminé Y5 de cycles respiratoires suivant la cinquième commande C5 d'augmentation de pression, on commande C6 une sixième augmentation prédéterminée de pression ;
- après la fin d'un sixième nombre prédéterminé Y6 de cycles respiratoires, supérieur au cinquième nombre Y5, suivant la cinquième commande C5 d'augmentation de pression, on commande par une commande C7 une septième augmentation de pression.

Le compteur de temps en hypopnée CTHO étant initialisé à 0 lors de la mise en marche de l'appareil.

Dans une réalisation, le quatrième nombre déterminé Y4 de cycles respiratoires de calcul d'amplitude moyenne est sensiblement égal à 8.

Le premier facteur prédéterminé FHO d'hypopnée est compris entre 1 et 100 % et est par exemple sensiblement égal à 40 %.

Le temps minimum d'hypopnée TMHO est compris entre 1 s et 25 s et est par exemple sensiblement égal à 10 s.

Les cinquième et sixième nombres prédéterminés Y5 ; Y6 de cycles respiratoires sont sensiblement égaux à respectivement 2 et 4.

La cinquième augmentation C5 prédéterminée de pression est comprise entre 0,1 mbar et 10 mbar et est par exemple sensiblement égale à + 1 mbar.

Les sixième et septième augmentations C6 ; C7 prédéterminées de pression sont chacune inférieures à la cinquième commande C5 et sont par exemple chacune sensiblement égales à la moitié de la cinquième augmentation C5 de pression.

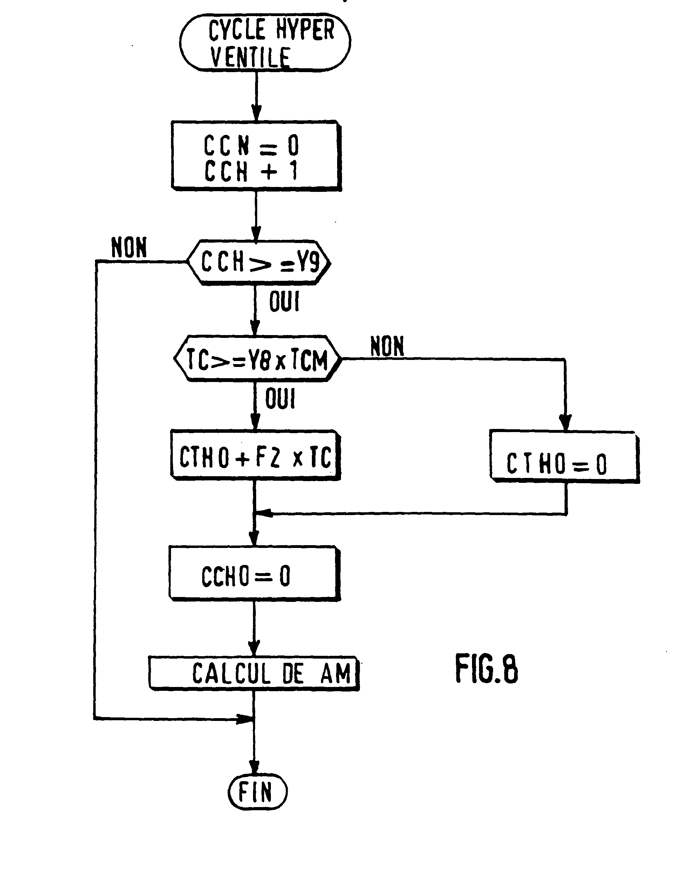

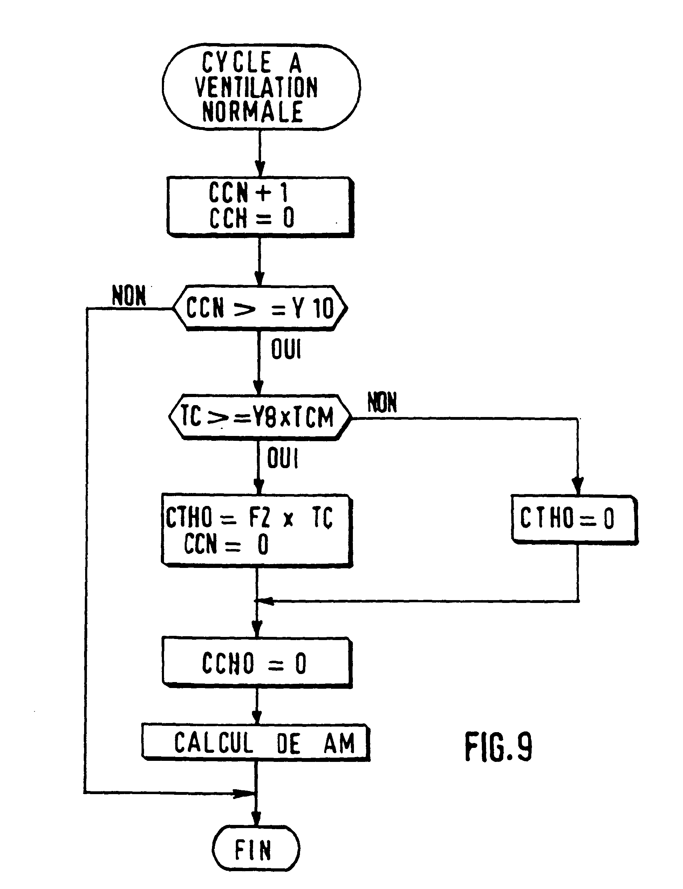

Comme représenté aux figures 8 et 9, si l'amplitude mesurée du dernier cycle respiratoire est supérieure ou égale à l'amplitude moyenne AM calculée multipliée par la premier facteur d'hypopnée FHO, alors on calcule le temps TCM de cycles respiratoires moyen sur un septième nombre prédéterminé Y7 de cycles précédents.

Si la durée mesurée TC du dernier cycle est supérieure à un huitième nombre prédéterminé Y8 multiplié par le temps de cycle respiratoire moyen calculé TCM, on ajoute au compteur de temps en hypopnée CTHO la durée mesurée TC du dernier cycle, multipliée par un deuxième facteur F2 d'hypopnée.

Si l'amplitude mesurée du dernier cycle respiratoire mesuré est supérieure à un troisième facteur F3 d'hyperventilation, supérieur au premier facteur FHO d'hypopnée, multiplié par l'amplitude moyenne calculée AM, on qualifie le dernier cycle d'hyperventilé, on incrémente d'une unité un compteur de cycles hyperventilés CCH, on remet à 0 un compteur CCN de cycles à ventilation normale et
■ si la valeur du compteur CCH de cycles hyperventilés est supérieure ou égale à un neuvième nombre prédéterminé Y9,
   ■ si la durée du dernier cycle TC est supérieure ou égale au huitième nombre Y8 multiplié par le temps de cycle moyen calculé TCM, on ajoute au compteur CTHO de temps en hypopnée le deuxième facteur F2 multiplié par la durée du dernier cycle respiratoire TC,
   ■ et sinon, on remet à 0 le compteur CTHO de temps en hypopnée ;
puis on remet à 0 un compteur CCHO de cycles hypoventilés et on calcule l'amplitude moyenne AM de cycle respiratoire sur le nombre prédéterminé Y4 de cycles respiratoires précédents.

Si l'amplitude mesurée du dernier cycle respiratoire mesuré est inférieure ou égale au troisième facteur F3 multiplié par l'amplitude moyenne calculée AM, on qualifie le dernier cycle de cycle à ventilation normale, on remet à 0 le compteur CCH de cycles hyperventilés et on incrémente d'une unité le compteur CCN de cycles à ventilation normale, et
■ si la valeur du compteur CCN de cycles à ventilation normale est supérieure ou égale à un dixième nombre Y10 prédéterminé.
   ■ si la durée du dernier cycle TC est supérieure ou égale au huitième nombre Y8 multiplié par le temps de cycle moyen calculé TCM, on affecte au compteur CTHO de temps en hypopnée le deuxième facteur F2 multiplié par la durée du dernier cycle TC et on remet à 0 le compteur CCN de cycle à ventilation normale,
   ■ et sinon, on remet à 0 le compteur CTHO de temps en hypopnée ;
puis on remet à 0 le compteur CCHO de cycles hypoventilés et on calcule l'amplitude moyenne du cycle respiratoire sur le nombre prédéterminé Y4 de cycles respiratoires.

Dans une réalisation, le deuxième facteur F2 est sensiblement égal à 5/8.

Le troisième facteur d'hyperventilation F3 est compris entre 100 % et 200 % et est par exemple sensiblement égal à 140 %.

Les septième, huitième, neuvième et dixième nombres prédéterminés Y7; Y8; Y9; Y10 sont respectivement sensiblement égaux à 3 ; 2 ; 2 ; et 2.

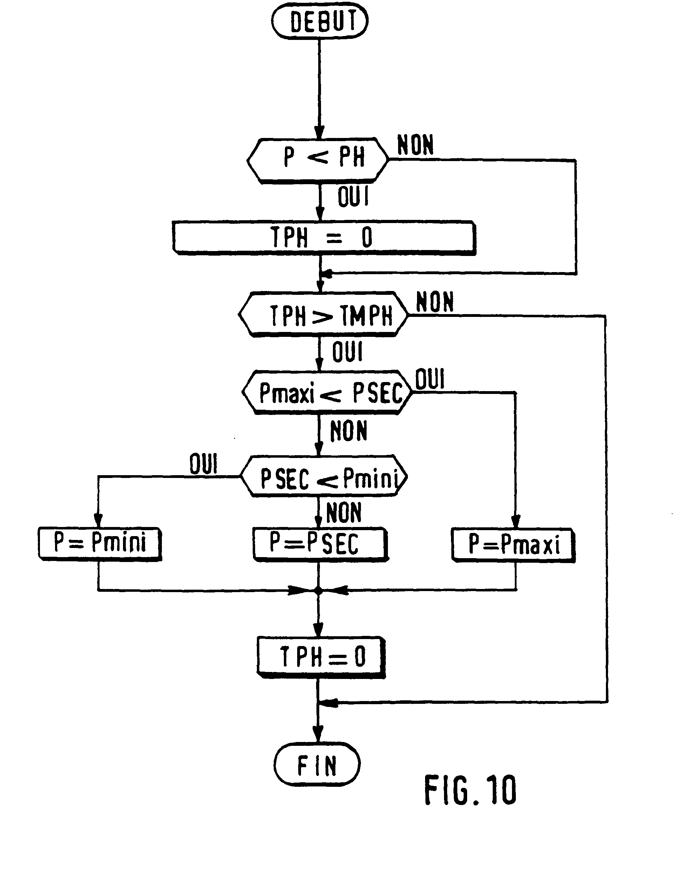

A la figure 10, on détecte si la pression est trop élevée.

Si la pression mesurée P est inférieure à une valeur de pression haute PH prédéterminée, on remet à 0 un compteur de temps en pression haute TPH.

Si la valeur du compteur de temps en pression haute TPH est supérieure à un temps maximum de pression haute TMPH et.
- si la valeur maximale de pression réglée Pmaxi est inférieure à une valeur de pression de sécurité PSEC prédéterminée, on commande la pression P à cette valeur maximale de pression réglée Pmaxi;
- si la valeur minimale de pression réglée Pmini est supérieure à une valeur de pression de sécurité PSEC prédéterminée, on commande la pression P à cette valeur minimale de pression réglée Pmini;
- si les deux précédentes conditions ne sont pas réalisées, on commande la pression P à la valeur de pression de sécurité PSEC,
puis
- on remet à 0 le compteur de temps en pression haute TPH.

Dans une réalisation, la valeur de pression haute PH est comprise entre 10 mbar et 25 mbar et est par exemple sensiblement égale à 17 mbar.

Le temps maximum de pression haute TMPH est compris entre 1 et 100 minutes et est par exemple sensiblement égal à 10 minutes ou 30 minutes.

La valeur de pression de sécurité PSEC est sensiblement égale à 8 mbar.

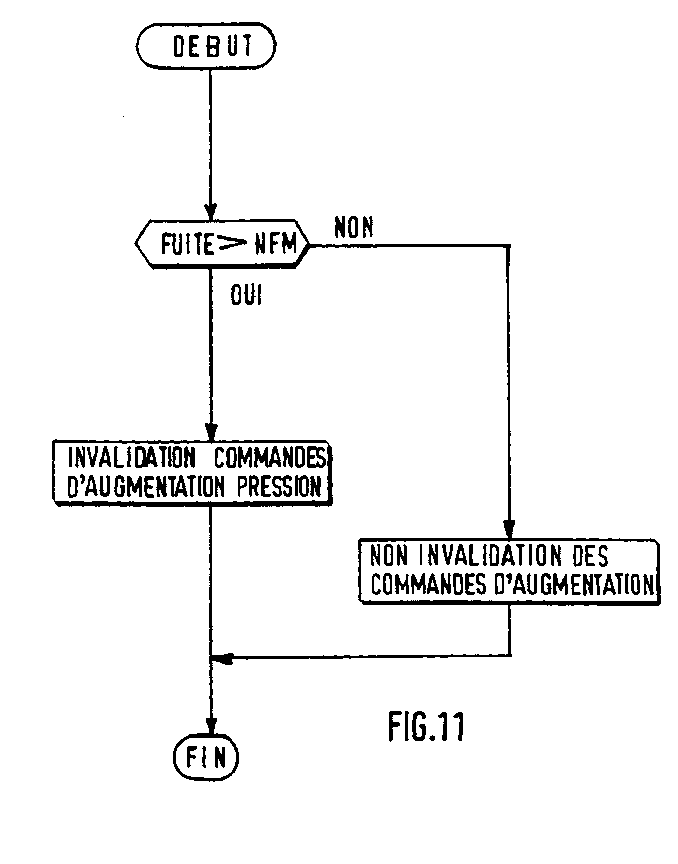

A la figure 11, on mesure une fuite d'air, sensiblement égale au débit moyen pendant la respiration du patient.

Si la fuite mesurée d'air est supérieure à un niveau prédéterminé de fuite NFM, on invalide les commandes d'augmentation de pression.

Dans une réalisation, NFM = A x Pfiltrée + B.

Selon cette formule, le niveau prédéterminé de fuite NFM est sensiblement égal à un coefficient A de fuite multiplié par une pression d'air filtrée dans le masque, ajouté à un coefficient B additif de fuite, le coefficient A de fuite étant compris entre 0 et 10 I/minute.mbar et étant par exemple sensiblement égal à 2,5 l/minute.mbar.

Le coefficient B additif de fuite est compris entre 0 et 100 l/mn et est par exemple sensiblement égal à 50 l/mn.

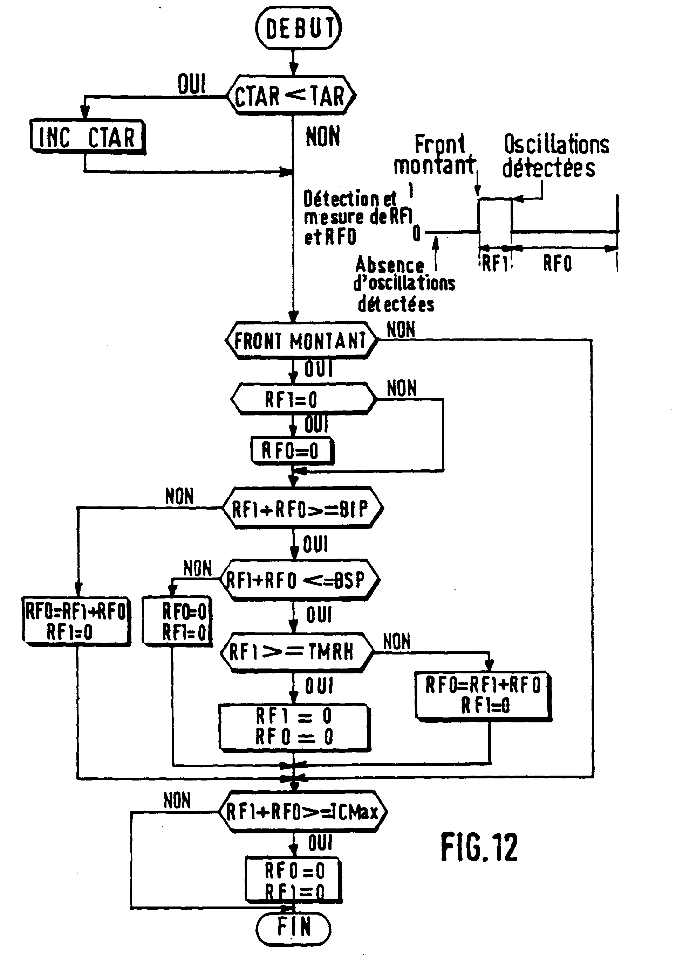

A la figure 12, on détecte si la courbe de pression mesurée présente des oscillations, telles que de vibrations acoustiques, comprises dans une plage P1 de fréquence.

Cette détection est effectuée par exemple par des moyens matériels tels que filtres analogiques ou numériques.

On mesure le temps RF1 de présence d'oscillations détectées entre deux absences successives d'oscillations détectées et le temps RF0 d'absence d'oscillations détectées entre deux présences successives d'oscillations détectées ;

Si la somme des temps mesurés d'absence et de présence d'oscillations détectées RF0 ; RF1 est comprise dans une plage temporelle prescrite BIP ; BSP.

Si le temps de présence d'oscillations RF1 mesuré est supérieur ou égal à un temps minimum d'oscillations TMRH et si la valeur d'un compteur CTAR de temps écoulé depuis l'avant-demière fois que les conditions temporelles précédentes ont été réalisées, est supérieure à un temps d'attente prescrit TAR, on commande C8 une huitième augmentation prédéterminée de pression et on remet le compteur de temps écoulé CTAR à 0.

Les algorithmes de détection de vibrations acoustiques et de commande en cas de vibrations acoustiques sont mis en oeuvre à intervalles de temps prescrits, notamment régulièrement et par exemple toutes les 100ms.

Au début de l'algorithme de détection de vibrations acoustiques représenté à la figure 12, si la valeur du compteur CTAR de temps écoulé est inférieure au temps d'attente prescrit TAR, on incrémente ( INC CTAR) ce compteur de l'intervalle de temps prescrit mentionné ci-dessus.

Si la somme des temps mesurés de présence et d'absence d'oscillations détectées RF0 ; RF1 est en deçà de la plage temporelle prescrite BIP ; BSP ou si le temps de présence mesuré d'oscillations détectées RF1 est inférieur au temps minimum d'oscillations TMRH,
- on remplace le temps mesuré RF0 d'absence d'oscillations détectées par la somme des temps mesurés d'absence et de présence d'oscillations détectées RF0 ; RF1, puis
- on remet à 0 le temps mesuré de présence d'oscillations détectées RF1.

Si la somme des temps mesurés d'absence et de présence d'oscillations détectées RF0 ; RF1 est au-delà de la plage temporelle prédéterminée BIP ; BSP ou d'un temps maximal prédéterminé TCMax, on remet à 0 chacun des temps mesurés d'absence et de présence d'oscillations détectées RF0 ; RF1 .

Si les deux conditions mentionnées ci-dessus sur la somme des temps de présence et d'absence RF1. RF0 et sur le temps de présence RF1 ne sont pas réalisées, on remet à 0 chacun des temps mesurés d'absence et de présence d'oscillations détectées RF0 ; RF1.

Dans une réalisation, le temps maximal prédéterminé TCMax est sensiblement égal à deux fois le temps TCM de cycle respiratoire moyen sur les trois derniers cycles mesurés.

La plage temporelle prescrite BIP; BSP est sensiblement comprise entre 10 % et 120 % du temps de cycle moyen calculé TCM.

Le temps minimum d'oscillation TMRH est sensiblement égal à 7 % du temps de cycle moyen calculé TCM.

Le temps d'attente prescrit TAR est compris entre 1 et 30 minutes et est par exemple sensiblement égal à 1 minute.

La huitième commande C8 d'augmentation de pression est comprise entre 0,1 mbar et 10 mbar et est par exemple sensiblement égale à 1 mbar.

La plage P1 de fréquence de détection d'oscillations est comprise entre sensiblement 30 et 300 Hz.

On mémorise la chronologie des événements détectés et on relève, par exemple après une nuit, la chronologie mémorisée.

A cet effet, l'unité centrale U de l'appareil comporte une mémoire non représentée pouvant être écrite et lue avec la chronologie des évènements détectés.

Cette chronologie peut être visualisée par exemple sur un moniteur en lisant le contenu de la mémoire, par l'intermédiaire d'un ordinateur non représenté.

## Revendications

1. Appareil de fourniture de pression d'air à un patient souffrant de troubles du sommeil tels qu'apnée comportant une unité (U) centrale de traitement et de commande de pression, un module (MPD) commandé de fourniture de pression, un masque (MVA) pour voies aériennes supérieures du patient, un conduit (CF) de fourniture de pression d'air du module (MPD) au masque (MVA), un capteur (CDAF) de débit d'air fourni, relié à l'unité centrale (U) et un capteur (CPM) de pression dans le masque (MVA), relié à l'unité centrale (U), un logiciel étant intégré à l'unité centrale (U), le logiciel étant agencé pour
- mesurer la pression d'air dans le masque et le débit d'air fourni au masque ;
- déterminer à partir des variables mesurées, si des événements représentatifs de troubles du sommeil apparaissent ou non,
**caractérisé en ce que** le logiciel est agencé pour
- déterminer à partir des variables mesurées si le cycle respiratoire actuel du patient correspond à un cycle respiratoire valide prédéterminé ;
- mettre dans un premier état (ON) d'apparition de troubles un indicateur (BLN) d'apparition de troubles, si l'apparition de l'un ou plusieurs des événements représentatifs de troubles du sommeil est déterminée; et
- mettre l'indicateur (BLN) dans un deuxième état (OFF) d'absence de troubles, dans le cas contraire,
- compter un premier nombre (CCAR) de cycles respiratoires valides déterminés depuis la dernière commande de pression ;
- compter un troisième nombre (RC) de passages successifs de l'indicateur (BLN) du deuxième état (OFF) au premier état (ON);
- commander (C1) une première augmentation déterminée de pression d'air fourni, lorsque, à la fois :
• le cycle respiratoire actuel a été déterminé comme étant valide ;
• le premier nombre (CCAR) est supérieur à un premier nombre (RP) entier prédéterminé ;
• le troisième nombre (RC) est supérieur ou égal à un troisième nombre (X) entier prédéterminé.

2. Appareil selon la revendication 1, **caractérisé en ce que** le logiciel est agencé pour
- compter un deuxième nombre (CCON) de cycles respiratoires valides déterminés depuis le dernier passage de l'indicateur (BLN) au premier état (ON) ;
- commander (C1) la première augmentation déterminée de pression d'air fourni, lorsque, en plus :
• le deuxième nombre (CCON) correspond à un autre ou plusieurs deuxièmes nombres (N) entiers prédéterminés.

3. Appareil selon la revendication 2, **caractérisé en ce que** les deuxièmes nombres entiers (N) sont compris entre 1 et 300 et sont par exemple les trois premiers multiples d'un entier (N₀) déterminé.

4. Appareil selon la revendication 3, **caractérisé en ce que** les deuxièmes nombres entiers (N) sont respectivement 2, 4 et 6.

5. Appareil selon l'une quelconque des revendications 1 à 4, **caractérisé en ce que** le premier nombre entier (RP) prédéterminé est compris entre 1 et 255.

6. Appareil selon la revendication 5, **caractérisé en ce que** le premier nombre entier (RP) prédéterminé est égal a 10.

7. Appareil selon l'une quelconque des revendications 1 à 6, **caractérisé en ce que** le troisième nombre entier (X) prédéterminé est compris entre 1 et 100.

8. Appareil selon la revendication 7, **caractérisé en ce que** le troisième nombre entier (X) prédéterminé est égal à 1.

9. Appareil selon l'une quelconque des revendications. 1 à 8, **caractérisé en ce que** la première commande (C1) déterminée d'augmentation de pression est inférieure à + 10 mbar.

10. Appareil selon la revendication 9, **caractérisé en ce que** la première commande (C1) déterminée d'augmentation de pression est sensiblement égale à + 0,3 mbar.

11. Appareil selon l'une quelconque des revendications 1 à 10, **caractérisé en ce que** le logiciel est agencé pour remettre à 0 les premiers et troisièmes (CCAR ; RC) nombres de cycles respiratoires valides comptés et de passages comptés, après que le deuxième nombre (CCON) compté de cycles valides a atteint le plus grand des deuxièmes nombres (N) entiers prédéterminés et remettre à 0 le deuxième nombre compté (CCON) lorsque l'indicateur (BLN) passe du deuxième état (OFF) au premier état (ON).

12. Appareil selon l'une quelconque des revendications 1 à 11, **caractérisé en ce que** le cycle respiratoire valide prédéterminé correspond à un maximum de débit respiratoire supérieur à une valeur de débit prédéterminée telle que 50 ml/s, à un volume inspiratoire supérieur à une valeur de volume prédéterminée telle que 0,05 1 et à une absence de saturation lors de la détection de débit.

13. Appareil selon l'une quelconque des revendications 1 à 12, **caractérisé en ce que** le logiciel est agencé pour
- initialiser, lors de la mise en marche de l'appareil, une variable (ER) d'état à un troisième état d'absence (NIR) de traitement et l'indicateur (BLN) au deuxième état (OFF) ;
puis, séquentiellement,
- qualifier, à partir des variables mesurées, les cycles respiratoires comme appartenant à différentes catégories telles que cycle à débit limité, cycle intermédiaire, cycle normal et cycle non valide, correspondant chacune respectivement à des pondérations (RSV0, REV0; RSV1, REV1; RSV2, REV2; 0, 0);
- affecter les pondérations de la catégorie du cycle actuellement qualifié à des premier et deuxième accumulateurs-(SV ; EV) de pondération ;
- si le cycle qualifié appartient à la catégorie de cycle non valide, remettre la variable d'état (ER) au troisième état (NIR) et l'indicateur (BLN) au deuxième état (OFF) et initialiser un premier compteur (FLC) à une valeur prédéterminée ;
- si l'état de la variable d'état (ER) correspond au troisième état (NIR) :
• si la valeur d'un premier accumulateur (SV) est inférieure à une première valeur comparative, réinitialiser le compteur (FLC) à sa valeur prédéterminée ;
• si la valeur du premier accumulateur (SV) est supérieure à sa première valeur comparative, faire passer la variable d'état (ER) à un quatrième état (PR) de possibilité de traitement et on met l'indicateur (BLN) au deuxième état (OFF) ;
- si l'état de la variable (ER) d'état correspond au quatrième état (PR) et
• si la valeur du premier accumulateur (SV) est inférieure à sa première valeur comparative, on réinitialise le premier compteur (FLC) à sa valeur prédéterminée, remettre la variable d'état (ER) et l'indicateur (BLN) respectivement aux troisième et deuxième états (NIR ; OFF);
• si la valeur du premier accumulateur (SV) est supérieure à sa première valeur comparative, faire prendre au premier compteur (FLC) sa valeur précédente additionnée de la valeur du premier accumulateur (SV) et si alors la valeur du premier compteur (FLC) est supérieure ou égale à une butée haute (RMS) prédéterminée :
■ réinitialiser un deuxième compteur (NC) à une valeur prédéterminée :
■ faire passer la variable d'état (ER) à un cinquième état (IR) de traitement ; et
■ faire passer l'indicateur (BLN) au premier état (ON) ;
- si l'état de la variable d'état (ER) correspond au cinquième état (IR) de traitement :
• si la valeur du deuxième accumulateur (EV) est supérieure à une deuxième valeur comparative, faire prendre au deuxième compteur (NC) sa valeur précédente additionnée de la valeur du deuxième accumulateur (EV) et
■ si alors la valeur du deuxième compteur (NC) est supérieure ou égale à une butée basse (RME), remettre la variable d'état (ER) et l'indicateur (BLN) respectivement aux troisièmes et deuxièmes états (NIR ; OFF) et réinitialiser les premier et deuxième compteurs (FLC ; NC) à leurs valeurs respectives prédéterminées ;
■ ou sinon, faire passer l'indicateur (BLN) à son premier état (ON) ;
■ si la valeur du deuxième accumulateur (EV) est inférieure à sa deuxième valeur comparative, réinitialiser le deuxième compteur (NC) à sa valeur respective prédéterminée et faire passer l'indicateur (BLN) au premier état (ON).

14. Appareil selon la revendication 13, **caractérisé en ce que** les pondérations (RSV2 , REV2; RSV1, REV1; RSV0, REV0; 0 ,0) correspondant aux catégories de cycle normal, cycle intermédiaire, cycle à débit limité et cycle non valide, sont respectivement sensiblement égales à -1 ; 1 ; 5 et 0 pour le premier accumulateur (SV) et sont respectivement sensiblement égales à 1 ; -1 ; -1 et 0 pour le deuxième accumulateur (EV), les première et deuxième valeurs comparatives et les valeurs prédéterminées d'initialisation des premier et deuxième compteurs (FLC ; NC) sont chacune sensiblement égales à 0 et les butées haute et basse (RMS ; RME) sont respectivement sensiblement égales à 10 et 2.

15. Appareil selon l'une quelconque des revendications 1 à 14, **caractérisé en ce que**:
- le cycle respiratoire valide prédéterminé correspond à un maximum de débit inspiratoire supérieur à une valeur de débit prédéterminée telle que 50 ml/s, à un volume inspiratoire supérieur à une valeur de volume prédéterminée telle que 0,05 1, à une absence de saturation lors de la détection de débit, à un temps inspiratoire mesuré compris dans un intervalle prédéterminé tel que de 0,5 s à 6 s et à une durée mesurée de cycles respiratoires comprise dans un autre intervalle prédéterminé tel que de 1,5 s à 20 s;
si le cycle respiratoire mesuré est déterminé comme étant valide, alors
- le logiciel calcule une courbe sinusoïdale équivalente respectant des caractéristiques prédéterminées par rapport à la courbe inspiratoire du cycle inspiratoire mesuré ;
- le logiciel calcule un critère de surface CS proportionnel au rapport de l'aire délimitée par la courbe inspiratoire sur l'aire délimitée par la courbe sinusoïdale équivalente, chacune étant prise sur un même intervalle de temps, compris dans la phase inspiratoire du cycle respiratoire mesuré ;
- le logiciel calcule un critère de corrélation entre la courbe inspiratoire du cycle inspiratoire mesuré et la courbe sinusoïdale équivalente ;
- si le critère (CC) de corrélation calculé est supérieur ou égal à une première limite normale prédéterminée (LN), et si le critère de surface calculé (CS) est supérieur à une deuxième limite prédéterminée (LS) de surface, le logiciel qualifie le cycle respiratoire mesuré de normal et sinon, le logiciel le qualifie de cycle à débit limité ;
- si le cycle respiratoire mesuré a été qualifié de cycle à débit limité,
• si le critère de surface (CS) calculé est supérieur à une troisième limite prédéterminée (LE) d'expert, le logiciel requalifie le cycle respiratoire mesuré de normal.
• ou sinon,
■ si le critère de surface (CS) calculé est supérieur à une quatrième limite prédéterminée (LD) de débit, le logiciel requalifie le cycle respiratoire mesuré d'intermédiaire,
■ et dans le cas contraire, le logiciel le qualifie de cycle à débit limité,
la deuxième limite (LS) de surface, la quatrième limite (LD) de débit et la troisième limite (LE) d'expert étant prédéterminées dans un ordre croissant

16. Appareil selon la revendication 15, **caractérisé en ce que** :
- les caractéristiques prédéterminées de la courbe sinusoïdale équivalente comprennent une demi-période sensiblement égale au temps inspiratoire mesuré et une pente à l'origine sensiblement égale à celle de la courbe inspiratoire lorsqu'elle atteint sensiblement un tiers de son amplitude maximale ;
- le critère de surface calculé (CS) est sensiblement égal à cent fois le rapport des aires prises chacune de sensiblement un quart à trois quart de la durée de la phase inspiratoire du cycle respiratoire mesuré ;
- le critère de corrélation calculé (CC) est sensiblement égal au maximum de cent fois les coefficients de corrélation entre la courbe inspiratoire et la courbe sinusoïdale équivalente prises respectivement sur la seconde moitié de la phase inspiratoire et sur la totalité de celle-ci ;
- les première, deuxième, quatrième et troisième limites (LN ; LS ; LD ; LE) étant respectivement comprises entre 45 et 100 ; 0 et 100 ; 0 et 100 ; 0 et 100 et étant par exemple sensiblement égales à 87 ; 40 ; 60 et 90 respectivement

17. Appareil selon l'une quelconque des revendications 1 à 16, **caractérisé en ce que** le logiciel est agencé pour
- calculer à chaque fin de cycle respiratoire mesuré, l'amplitude moyenne (AM) sur un quatrième nombre prédéterminé (Y4) de cycles respiratoires précédents,
- si l'amplitude mesurée du dernier cycle respiratoire est inférieure à l'amplitude moyenne (AM) calculée multipliée par un premier facteur d'hypopnée prédéterminé (FHO), alors ajouter à un compteur (CTHO) de temps en hypopnée la durée (TC) du dernier cycle respiratoire mesuré,
• si la valeur actuelle du compteur de temps en hypopnée (CTHO) est supérieure ou égale à un temps minimum d'hypopnée (TMHO), commander (C5) une cinquième augmentation prédéterminée de pression,
• après la fin d'un cinquième nombre prédéterminé (Y5) de cycles respiratoires suivant la cinquième commande (C5) d'augmentation de pression, commander (C6) une sixième augmentation prédéterminée de pression ;
• après la fin d'un sixième nombre prédéterminé (Y6) de cycles respiratoires, supérieur au cinquième nombre (Y5), suivant la cinquième commande (C5) d'augmentation de pression, commander (C7) une septième augmentation de pression,
- le compteur de temps en hypopnée (CTHO) étant initialisé à 0 lors de la mise en marche de l'appareil.

18. Appareil selon la revendication 17, **caractérisé en ce que** :
- le quatrième nombre déterminé (Y4) de cycles respiratoires de calcul d'amplitude moyenne est sensiblement égal à 8,
- le premier facteur prédéterminé (FHO) d'hypopnée est compris entre 1 et 100 % et est par exemple sensiblement égal à 40 %,
le temps minimum d'hypopnée (TMHO) est compris entre 1 s et 25 s et est par exemple sensiblement égal à 10 s,
les cinquième et sixième nombres prédéterminés (Y5 ; Y6) de cycles respiratoires sont sensiblement égaux à respectivement 2 et 4,
la cinquième augmentation (C5) prédéterminée de pression est comprise entre 0,1 mbar et 10 mbar et est par exemple sensiblement égale à + 1 mbar,
les sixième et septième augmentations (C6 ; C7) prédéterminées de pression étant chacune inférieures à la cinquième commande (C5) et étant par exemple chacune sensiblement égales à la moitié de la cinquième augmentation (C5) de pression.

19. Appareil selon la revendication 18, **caractérisé en ce que** le logiciel est agencé pour
- si l'amplitude mesurée du dernier cycle respiratoire est supérieure ou égale à l'amplitude moyenne (AM) calculée multipliée par la premier facteur d'hypopnée (FHO), alors calculer le temps (TCM) de cycles respiratoires moyen sur un septième nombre prédéterminé (Y7) de cycles précédents ;
• si la durée mesurée (TC) du dernier cycle est supérieure à un huitième nombre prédéterminé (Y8) multiplié par le temps de cycle respiratoire moyen calculé (TCM), ajouter au compteur de temps en hypopnée (CTHO) la durée (TC) mesurée du dernier cycle, multipliée par un deuxième facteur (F2) d'hypopnée,
• si l'amplitude mesurée du dernier cycle respiratoire mesuré est supérieure à un troisième facteur (F3) d'hyperventilation, supérieur au premier facteur (FHO) d'hypopnée, multiplié par l'amplitude moyenne calculée (AM), qualifier le dernier cycle d'hyperventilé, incrémenter d'une unité un compteur de cycles hyperventilés (CCH), remettre à 0 un compteur (CCN) de cycles à ventilation normale et
• si la valeur du compteur (CCH) de cycles hyperventilés est supérieure ou égale à un neuvième nombre prédéterminé (Y9),
• si la durée du dernier cycle (TC) est supérieure ou égale au huitième nombre (Y8) multiplié par le temps de cycle moyen calculé (TCM), ajouter au compteur (CTHO) de temps en hypopnée le deuxième facteur (F2) multiplié par la durée du dernier cycle respiratoire (TC),
• et sinon, remettre à 0 le compteur (CTHO) de temps en hypopnée ;
puis remettre à 0 un compteur (CCHO) de cycles hypoventilés et on calcule l'amplitude moyenne (AM) de cycle respiratoire sur le nombre prédétermine (Y4) de cycles respiratoires précédents,
• si l'amplitude mesurée du dernier cycle respiratoire mesuré est inférieure ou égale au troisième facteur (F3) multiplié par l'amplitude moyenne calculée (AM), qualifier le dernier cycle de cycle à ventilation normale, remettre à 0 le compteur (CCH) de cycles hyperventilés et on incrémente d'une unité le compteur (CCN) de cycles à ventilation normale, et
■ si la valeur du compteur (CCN) de cycles à ventilation normale est supérieure ou égale à un dixième nombre (Y10) prédéterminé,
■ si la durée du dernier cycle (TC) est supérieure ou égale au huitième nombre (Y8) multiplié par le temps de cycle moyen calculé (TCM), affecter au compteur (CTHO) de temps en hypopnée le deuxième facteur (F2) multiplié par la durée du dernier cycle (TC) et on remet à 0 le compteur (CCN) de cycle à ventilation normale.
■ et sinon, remettre à 0 le compteur (CTHO) de temps en hypopnée ; puis remettre à 0 le compteur (CCHO) de cycles hypoventilés et calculer l'amplitude moyenne du cycle respiratoire sur le nombre prédéterminé (Y4) de cycles respiratoires.

20. Appareil selon la revendication 19, **caractérisé en ce que** le deuxième facteur (F2) est sensiblement égal à 5/8,
le troisième facteur d'hyperventilation (F3) est compris entre 100 % et 200 % et est par exemple sensiblement égal à 140 %,
les septième, huitième, neuvième et dixième nombres prédéterminés (Y7 ; Y8 ; Y9 ; Y10) sont respectivement sensiblement égaux à 3 ; 2 ; 2 ; et 2.

21. Appareil selon l'une quelconque des revendications 1 à 20, **caractérisé en ce que** le logiciel est agencé pour
- si la pression mesurée (P) est inférieure à une valeur de pression haute (PH) prédéterminée, remettre à 0 un compteur de temps en pression haute (TPH) ;
- si la valeur du compteur de temps en pression haute (TPH) est supérieure à un temps maximum de pression haute (TMPH) et
• si la valeur maximale de pression réglée (Pmaxi) est inférieure à une valeur de pression de sécurité (PSEC) prédéterminée, commander la pression (P) à cette valeur maximale de pression réglée (Pmaxi);
• si la valeur minimale de pression réglée (Pmini) est supérieure à une valeur de pression de sécurité (PSEC) prédéterminée, commander la pression (P) à cette valeur minimale de pression réglée (Pmini);
• si les deux précédentes conditions ne sont pas réalisées , commander la pression (P) à la valeur de pression de sécurité (PSEC) ;
puis
• remettre à 0 le compteur de temps en pression haute (TPH).

22. Appareil selon la revendication 21, **caractérisé en ce que** la valeur de pression haute (PH) est comprise entre 10 mbar et 25 mbar et est par exemple sensiblement égale à 17 mbar,
le temps maximum de pression haute (TMPH) est compris entre 1 et 100 minutes et est par exemple sensiblement égal à 10 minutes ou 30 minutes,
la valeur de pression de sécurité (PSEC) est sensiblement égale à 8 mbar.

23. Appareil selon l'une quelconque des revendications 1 à 22, **caractérisé en ce que** le logiciel est agencé pour mesurer une fuite d'air, sensiblement égale au débit moyen pendant la respiration du patient,
- si la fuite-mesurée d'air est supérieure à un niveau prédéterminé de fuite (NFM), invalider les commandes d'augmentation de pression.

24. Appareil selon la revendication 23, **caractérisé en ce que** le niveau prédéterminé de fuite (NFM) est sensiblement égal à un coefficient (A) de fuite multiplié par une pression d'air filtrée dans le masque, ajouté à un coefficient (B) additif de fuite, le coefficient (A) de fuite étant compris entre 0 et 10 I/minute.mbar et étant par exemple sensiblement égal à 2,5 I/minute.mbar, et
le coefficient (B) additif de fuite est compris entre 0 et 100 I/mn et est par exemple sensiblement égal à 50 l/mn.

25. Appareil selon l'une quelconque des revendications 1 à 24, **caractérisé en ce que** le logiciel est agencé pour
- détecter si la courbe de pression mesurée présente des oscillations, telles que de vibrations acoustiques, comprises dans une plage (P1) de fréquence,
- mesurer le temps (RF1) de présence d'oscillations détectées entre deux absences successives d'oscillations détectées et le temps (RF0) d'absence d'oscillations détectées entre deux présences successives d'oscillations détectées ;
- si la somme des temps mesurés d'absence et de présence d'oscillations détectées (RF0 ; RF1) est comprise dans une plage temporelle prescrite (BIP ; BSP),
- si le temps de présence d'oscillations (RF1) mesuré est supérieur ou égal à un temps minimum d'oscillations (TMRH) et
- si la valeur d'un compteur (CTAR) de temps écoulé depuis l'avant-demière fois que les conditions temporelles précédentes ont été réalisées, est supérieure à un temps d'attente prescrit (TAR), commander (C8) une huitième augmentation prédéterminée de pression et remettre le compteur de temps écoulé (CTAR) à 0.

26. Appareil suivant la revendication 25, **caractérisé en ce que** le logiciel est agencé pour
- si la somme des temps mesurés de présence et d'absence d'oscillations détectées (RF0 ; RF1) est en deçà de la plage temporelle prescrite (BIP ; BSP) ou si le temps de présence mesuré d'oscillations détectées (RF1) est inférieur au temps minimum d'oscillations (TMRH),
• remplacer le temps mesuré (RF0) d'absence d'oscillations détectées par la somme des temps mesurés d'absence et de présence d'oscillations détectées (RF0 ; RF1), puis
• remettre à 0 le temps mesuré de présence d'oscillations détectées (RF1), et
- si la somme des temps mesurés d'absence et de présence d'oscillations détectées (RFO ; RF1) est au-delà de la plage temporelle prédéterminée (BIP ; BSP) ou d'un temps maximal prédéterminé (TCMax), remettre à 0 chacun des temps mesurés d'absence et de présence d'oscillations détectées (RFO ; RF1) ; et sinon
- remettre à 0 chacun des temps mesurés d'absence et de présence d'oscillations détectées (RF0 ; RF1).

27. Appareil selon l'une quelconque des revendications 25 et 26, **caractérisé en ce que** :
le temps maximal prédéterminé est sensiblement égal à deux fois le temps (TQM) de cycle respiratoire moyen sur les trois derniers cycles mesurés ;
- la plage temporelle prescrite (BIP; BSP) est sensiblement comprise entre 10 % et 120 % du temps de cycle moyen calculé (TCM) ;
- le temps minimum d'oscillation (TMRH) est sensiblement égal à 7 % du temps de cycle moyen calculé (TCM) ;
- le temps d'attente prescrit (TAR) est compris entre 1 et 30 minutes et est par exemple sensiblement égal à 1 minute ;
- la huitième commande (C8) d'augmentation de pression est comprise entre 0,1 mbar et 10 mbar et est par exemple sensiblement égale à 1 mbar ;
- la plage (P1) de fréquence de détection d'oscillations est comprise entre sensiblement 30 et 300 Hz.

28. Appareil suivant l'une quelconque des revendications 1 à 27, **caractérisé en ce que** logiciel est agencé pour mémoriser la chronologie des événements détectés et on relève, par exemple après une nuit, la chronologie mémorisée.

29. Appareil selon l'une quelconque des revendications 1 à 28 **caractérisé en ce qu'**il comporte une mémoire de la chronologie des événements détectés, dont le contenu est apte à être relevé, par exemple après une nuit.

## Claims

1. Apparatus for supplying air pressure to a patient suffering from sleep disorders such as apnea, said apparatus comprising a pressure-control and central processing unit (U), a controlled pressure supply module, an upper airway mask (MVA) for the upper airways of the patient, an air pressure supply duct (CF) for supplying air pressure from the module (MPD) to the mask (MVA), a supplied air flow rate sensor (CDAF) connected to the central processing unit (U) and a pressure sensor (CPM) for sensing the pressure in the mask (MVA), which pressure sensor is connected to the central processing unit (U), a piece of software being incorporated into the central processing unit (U), the piece of software being arranged:
- to measure the air pressure in the mask and the flow rate of air supplied to the mask; and
- on the basis of the measured variations, to determine whether or not events representative of sleep apnea appear;
said apparatus being **characterized in that** the software is arranged:
- on the basis of the measured variations, to determine whether the current breathing cycle of the patient corresponds to a predetermined valid breathing cycle;
- to put a disorder appearance indicator (BLN) in a disorder-appearance first state (ON) if it is determined that one or more events representative of sleep disorders have appeared;
- to put the indicator (BLN) in an absence-of-disorder second state (OFF) otherwise;
- to count a first number (CCAR) of valid breathing cycles determined since the last pressure instruction;
- to count a third number (RC) of successive changes of state during which the indicator (BLN) goes over from the second state (OFF) to the first state (ON);
- to issue an instruction (C1) for causing a first determined increase in supplied air pressure when, at the same time:
• the current breathing cycle is determined as being valid;
• the first number (CCAR) is greater than a first predetermined integer number (RP); and
• the third number (RC) is greater than or equal to a third predetermined integer number (X).

2. Apparatus according to claim 1, **characterized in that** the piece of software is arranged:
- to count a second number (CCON) of valid breathing cycles determined from the last change of state during which the indicator (BLN) went over to the first state (ON);
- to issue an instruction (C1) for causing the first determined increase in supplied air pressure when, in addition:
• the second number (CCON) corresponds to one or more other second predetermined integer numbers (N).

3. Apparatus according to claim 2, **characterized in that** the second integer numbers (N) lie in the range 1 to 300 and are, for example, the first three multiples of a determined integer (N₀).

4. Apparatus according to claim 3, **characterized in that** the second integer numbers (N) are respectively 2, 4, and 6.

5. Apparatus according to any one of claims 1 to 4, **characterized in that** the first predetermined integer number (RP) lies in the range 1 to 255;

6. Apparatus according to claim 5, **characterized in that** the first predetermined integer number (RP) is equal to 10.

7. Apparatus according to any one of claims 1 to 6, **characterized in that** the third predetermined integer number (X) lies in the range 1 to 100.

8. Apparatus according to claim 7, **characterized in that** the third predetermined integer number (X) is equal to 1.

9. Apparatus according to any one of claims 1 to 8, **characterized in that** the first determined pressure-increase instruction (C1) is for a pressure increase of less than +10 mbar.

10. Apparatus according to claim 9, **characterized in that** the first determined pressure-increase instruction (C1) is for a pressure increase equal to +0.3 mbar.

11. Apparatus according to any one of claims 1 to 10, **characterized in that** the piece of software is arranged to reset the first and third numbers (CCAR; RC) of valid breathing cycles counted and of changes of state counted, after the counted second number (CCON) of valid cycles has reached the largest of the second predetermined integer numbers (N), and to reset the counted second number (CCON) when the indicator (BLN) goes from the second state (OFF) to the first state (ON).

12. Apparatus according to any one of claims 1 to 11, **characterized in that** the predetermined valid breathing cycle corresponds to a maximum breathing flow rate greater than a predetermined flow-rate value such as 50 ml/s, to an inhalation volume greater than a predetermined volume such as 0.05 1, and to an absence of saturation when the flow rate is detected.

13. Apparatus according to any one of claims 1 to 12, **characterized in that** the piece of software is arranged:
- on switching on the apparatus, to initialize a state variable (ER) to an absence-of-processing third state (NIR) and to initialize the indicator (BLN) to the second state (OFF);
then, sequentially:
- on the basis of the measured variables, to qualify the breathing cycles as belonging to respective categories such as limited flow rate cycle, intermediate cycle, normal cycle, and invalid cycle, each of which corresponds to respective weightings (RSV0, REV0; RSV1, REV1; RSV2, REV2; 0,0);
- to assign the weightings of the category of the currently qualified cycle to first and second weighting accumulators (SV; EV);
- if the qualified cycle belongs to the invalid cycle category, to change the state variable (ER) back to the third state (NIR) and to change the indicator (BLN) back to the second state (OFF), and to initialize a first counter (FLC) to a predetermined value;
- if the state variable (ER) corresponds to the third state (NIR):
• if the value of a first accumulator (SV) is less than a first comparative value, to reinitialize the counter (FLC) to its predetermined value; and
• if the value of the first accumulator (SV) is greater than its first comparative value, to cause the state variable (ER) to go to a processing possibility fourth state (PR), and to put the indicator (BLN) into the second state (OFF);
- if the state variable (ER) corresponds to the fourth state (PR); and
• if the value of the first accumulator (SV) is less than its first comparative value, to reinitialize the first counter (FLC) to its predetermined value, to change the state variable (ER) and the indicator (BLN) back respectively to the third and to the second states (NIR; OFF);
• if the value of the first accumulator (SV) is greater than its first comparative value, to cause the first counter (FLC) to take its preceding value added to the value of the first accumulator (SV), and if the value of the first counter (FLC) is then greater than or equal to a predetermined upper limit (RMS):
• to reinitialize a second counter (NC) to a predetermined value;
• to change the state variable (ER) over to a processing fifth state (IR); and
• to change the indicator (BLN) over to the first state (ON);
- if the state variable (ER) corresponds to the processing fifth state (IR):
• if the value of the second accumulator (EV) is greater than a second comparative value, to cause the second counter (NC) to take its preceding value added to the value of the second accumulator (EV); and
• if the value of the second counter (NC) is then greater than or equal to a lower limit (RME), to put the state variable (ER) and the indicator (BLN) back respectively to the third and to the second states (NIR; OFF), and to reinitialize the first and second counters (FLC; NC) to their respective predetermined values;
• or otherwise, to change the indicator (BLN) over to its first state (ON);
• if the value of the second accumulator (EV) is less than its second comparative value, to reinitialize the second counter (NC) to its respective predetermined value, and to change the indicator (BLN) over to the first state (ON).

14. Apparatus according to claim 13, **characterized in that** the weightings (RSV2, REV2; RSV1, REV1; RSV0, REV0; 0, 0) corresponding to the categories of normal cycle, intermediate cycle, limited flow rate cycle, and invalid cycle, are respectively substantially equal to -1; 1; 5; and 0 for the first accumulator (SV) and are respectively substantially equal to 1; -1; -1; and 0 for the second accumulator (EV), the first and second comparative values and the predetermined values for initializing the first and second counters (FLC; NC) are each substantially equal to 0, and the upper and lower limits (RMS; RME) are respectively substantially equal to 10 and 2.

15. Apparatus according to any one of claims 1 to 14, **characterized in that**:
- the predetermined valid breathing cycle corresponds to a maximum inhalation flow rate greater than a predetermined flow rate such as 50 ml/s, to an inhalation volume greater than a predetermined volume value such as 0.05 1, to an absence of saturation when the flow rate is detected, to a measured inhalation time lying in a predetermined range such as 0.5 seconds (s) to 6 s, and to a measured breathing cycle time lying in another predetermined range such as 1.5 s to 20 s;
if the measured breathing cycle is determined as being valid; then
- the piece of software computes an equivalent sinewave curve complying with the predetermined characteristics relative to the inhalation curve of the measured inhalation cycle;
- the piece of software computes an area criterion CS that is proportional to the ratio of the area defined by the inhalation curve to the area defined by the equivalent sinewave curve; each being taken over the same time interval, lying within the inhalation stage of the measured breathing cycle;
- the piece of software computes a correlation criterion (CC) for correlation between the inhalation curve of the measured inhalation cycle and the equivalent sinewave curve;
- if the computed correlation criterion (CC) is greater than or equal to a normal first predetermined limit (LN), and if the computed area criterion (CC) is greater than an area second predetermined limit LS), the piece of software qualifies the measured breathing cycle as normal, and, otherwise, the piece of software qualifies it as a limited flow rate cycle;
- if the measured breathing cycle is qualified as a limited flow rate cycle;
• if the computed area criterion (CS) is greater than an expert third predetermined limit, the piece of software requalifies the measured breathing cycle as a normal cycle;
• or otherwise:
• if the computed area criterion (CS) is greater than a flow-rate fourth predetermined limit (LD), the piece of software requalifies the measured breathing cycle as an intermediate cycle; and
• otherwise, the piece of software qualifies it as a limited flow rate cycle;
the area second limit (LS), the flow rate fourth limit (LD), and the expert third limit (LE) being predetermined in increasing order.

16. Apparatus according to claim 15, **characterized in that**:
- the predetermined characteristics of the equivalent sinewave curve comprise a half-period substantially equal to the measured inhalation time, and a gradient at the origin substantially equal to the gradient at the origin of the inhalation curve when it reaches substantially one third of its maximum amplitude;
- the computed area criterion (CS) is substantially equal to one hundred times the ratio of the areas, each of which is taken from substantially one quarter to three quarters of the time of the inhalation stage of the measured breathing cycle;
- the computed correlation cycle (CC) is substantially equal to a maximum of one hundred times the correlation coefficients for correlation between the inhalation curve and the equivalent sinewave curve, taken respectively over the second half of the inhalation stage and over the entire inhalation stage; and
- the first, second, fourth, and third limits (LN; LS; LD; LE) respectively lying in the range 45 to 100; 0 to 100; 0 to 100; and 0 to 100, and being, for example, substantially equal to 87; 40; 60; and 90, respectively.

17. Apparatus according to any one of claims 1 to 16, **characterized in that** the piece of software is arranged:
- at the end of each measured breathing cycle, to compute the mean amplitude (AM) over a predetermined fourth number (Y4) of preceding breathing cycles;
- if the measured amplitude of the last breathing cycle is less than the computed mean amplitude (AM) multiplied by a hypopnea first predetermined factor (FHO), then to add the time (TC) of the last measured breathing cycle to a hypopnea time timer (CTHO);
• if the current value of the hypopnea time timer (CTHO) is greater than or equal to a minimum hypopnea time (TMHO), to issue an instruction (C5) for causing a fifth predetermined pressure increase;
• after a fifth predetermined number (Y5) of breathing cycles after the fifth pressure-increase instruction (C5), to issue an instruction (C6) for causing a sixth predetermined pressure increase;
• after a sixth predetermined number (Y6) of breathing cycles, which number is larger than the fifth number (Y5), after the fifth pressure-increase instruction (C5), to issue a seventh pressure-increase instruction (C7);
- the hypopnea time timer (CTHO) being initialized to 0 on switching on the apparatus.

18. Apparatus according to claim 17, **characterized in that**:
- the fourth determined number (Y4) of breathing cycles for computing the mean amplitude is substantially equal to 8;
- the hypopnea first predetermined factor (FHO) lies in the range 1% to 100% and is, for example, substantially equal to 40%;
- the minimum hypopnea time (TMHO) lies in the range 1 s to 25 s and is, for example, substantially equal to 10 s;
- the fifth and sixth predetermined numbers (Y5; Y6) of breathing cycles are substantially equal respectively to 2 and to 4;
- the fifth predetermined pressure increase (C5) lies in the range 0.1 mbar to 10 mbar, and is, for example, substantially equal to +1 mbar; and
- each of the sixth and seventh predetermined pressure increases (C8; C7) being less than the pressure increase of the fifth instruction (C5) and, for example, each being substantially equal to one half of the fifth pressure increase (C5).

19. Apparatus according to claim 18, **characterized in that** the piece of software is arranged:
- if the measured amplitude of the last breathing cycle is greater than or equal to the computed mean amplitude (AM) multiplied by the hypopnea first factor (FHO), then to compute the mean breathing cycle time (TCM) over a seventh predetermined number (Y7) of preceding cycles;
• if the measured time (TC) of the last cycle is greater than an eighth predetermined number (Y8) multiplied by the computed mean breathing cycle (TCM), to add the measured time (TC) of the last cycle, multiplied by a hypopnea second factor (F2), to the hypopnea time timer (CTHO);
• if the measured amplitude of the last breathing cycle is greater than a hyperventilation third factor (F3), greater than the hypopnea first factor (FHO), multiplied by the computed mean amplitude (AM), to qualify the last cycle as a hyperventilated cycle, to increment by one unit a hyperventilated cycle counter (CCH), and to reset a normal ventilation cycle counter (CCN); and
• if the value of the hyperventilated cycle counter (CCH) is greater than or equal to a ninth predetermined number (Y9);
• if the time of the last cycle (TC) is greater than or equal to the eighth number (Y8) multiplied by the computed mean cycle time (TCM), to add the second factor (F2 multiplied by the time of the last breathing cycle (TC) to the hypopnea time timer (CTHO);
• and otherwise to reset the hypopnea time timer (CTHO);
then to reset a hypoventilated cycle counter (CCHO) and to compute the mean breathing cycle amplitude (AM) over the predetermined number (Y4) of preceding breathing cycles;
• if the measured amplitude of the last breathing cycle measured is less than or equal to the third factor (F3) multiplied by the computed mean amplitude (AM), to qualify the last cycle as a normal ventilation cycle, to rest the hyperventilated cycle counter (CCH), and to increment the normal ventilation cycle counter (CCN) by one unit; and
• if the normal ventilation cycle counter (CCN) is greater than or equal to a tenth predetermined number (Y10);
• if the time of the last cycle (TC) is greater than or equal to the eighth number (Y8) multiplied by the computed mean cycle time (TCM), to assign to the hypopnea time timer (CTHO) the second factor (F2) multiplied by the time of the last cycle (TC), and to reset the normal ventilation cycle counter (CCN); and
• otherwise, to reset the hypopnea time timer (CTHO);
then to reset the hypoventilated cycle counter and to compute the mean amplitude of the breathing cycle over the predetermined number (Y4) of breathing cycles.

20. Apparatus according to claim 19, **characterized in that**:
- the second factor (F2) is substantially equal to 5/8;
- the hyperventilation third factor (F3) lies in the range 100% to 200% and is, for example, substantially equal to 140%; and
- the seventh, eighth, ninth, and tenth predetermined numbers (Y7; Y8; Y9; Y10) are respectively equal to 3; 2; 2; and 2.

21. Apparatus according to any one of claims 1 to 20, **characterized in that** the piece of software is arranged:
- if the measured pressure (P) is less than a predetermined high pressure value (PH), to reset a high pressure time timer (TPH;
• if the value of the high pressure time timer (TPH) is greater than a maximum high pressure time (TMPH); and
• if the maximum regulated pressure value (Pmaxi) is less than a predetermined safety pressure value (PSEC), to cause the pressure (P) to go to said maximum regulated pressure value (Pmaxi);
• if the minimum regulated pressure value (Pmini) is greater than a predetermined safety pressure value (PSEC), to cause the pressure (P) to go to said minimum regulated pressure value (Pmini);
• if both of the preceding conditions are not satisfied, to cause the pressure (P) to go to the safety pressure value (PSEC); then
• to reset the high pressure time timer (TPH).

22. Apparatus according to claim 21, **characterized in that**:
- the high pressure value (PH) lies in the range 10 mbar to 25 mbar and is, for example, substantially equal to 17 mbar;
- the maximum high pressure time (TMPH) lies in the range 1 minutes to 100 minutes, and is, for example, substantially equal to 10 minutes or to 30 minutes; and
- the safety pressure value (PSEC) is substantially equal to 8 mbar.

23. Apparatus according to any one of claims 1 to 22, **characterized in that** the piece of software is arranged:
- to measure an air leak substantially equal to the mean flow rate while the patient is breathing;
- if the measured air leak is greater than a predetermined leakage level (NFM), to invalidate the pressure-increase instructions.

24. Apparatus according to claim 23, **characterized in that**:
- the predetermined leakage level (NFM) is substantially equal to a leakage coefficient (A) multiplied by a filtered air pressure in the mask, added to an additional leakage coefficient (B), the leakage coefficient (A) lying in the range 0 1/minute.mbar to 10 1/minute.mbar, and being, for example, substantially equal to 2.5 1/minute.mbar; and
- the additional leakage coefficient (B) lies in the range 0 liters per minute (l/min) to 100 l/min and is, for example, substantially equal to 50 l/min.

25. Apparatus according to any one of claims 1 to 24, **characterized in that** the piece of software is arranged:
- to detect whether the measured pressure cure presents oscillations, such as acoustic vibrations, lying in a frequency range (P1);
- to measure the time (RF1) of presence of detected oscillations between two successive absences of detected oscillations, and the time (RF0) of absence of detected oscillations between two successive presences of detected oscillations;
- if the sum of the measured times of absence and of presence of detected oscillations (RF0; RF1) lies in a required time range (BIP; BSP);
- if the measured time of presence of oscillations (RF1) is greater than or equal to a minimum oscillation time (TMRH); and
- if the value of a timer (CTAR) for timing the time elapsed from the penultimate occasion on which the preceding time conditions were satisfied is greater than a required waiting time (TAR), to issue an instruction (C8) for an eighth predetermined increase in pressure, and to reset the elapsed time timer (CTAR).

26. Apparatus according to claim 25, **characterized in that** the piece of software is arranged:
- if the sum of the measured times of presence and of absence of detected oscillations (RF0; RF1) lies within the required time range (BIP; BSP), or if the measured time of detected oscillations (RF1) is less than the minimum oscillation time (TMRH);
• to replace the measured time (RFO) of absence of detected oscillations with the sum of the measured times of absence and of presence of detected oscillations (RFO; RF1); then
• to reset the measured time of presence of detected oscillations (RF1); and
- if the sum of the measured times of absence and of presence of detected oscillations (RF0; RF1) lies outside the predetermined time range (BIP; BSP) or of a predetermined maximum time (TCMax), to reset each of the measured times of absence and of presence of detected oscillations (RF0; RF1); and otherwise
- to reset each of the measured times of absence and of presence of detected oscillations (RF0; RF1).

27. Apparatus according to claim 25 or claim 26, **characterized in that**:
- the predetermined maximum time is substantially equal to twice the mean breathing cycle time (TCM) over the last three measured cycles;
- the required time range (BIP; BSP) substantially lies in the range 10% of the computed mean cycle time (TCM) to 120% of said computed mean cycle time;
- the minimum oscillation time (TMRH) is substantially equal to 7% of the computed mean cycle time (TCM);
- the required waiting time (TAR) lies in the range 1 minute to 30 minutes, and is, for example, substantially equal to 1 minute;
- the eighth instruction (C8) for increasing pressure is for a pressure increase lying in the range 0.1 mbar to 10 mbar and is, for example, substantially equal to 1 mbar; and
- the oscillation detection frequency range (P1) lies substantially in the range 30 Hz to 300 Hz.

28. Apparatus according to any one of claims 1 to 27, **characterized in that** the piece of software is arranged to store the chronology of the detected events and to enable a reading of the stored chronology to be taken, e.g. after one night.

29. Apparatus according to any one of claims 1 to 28, **characterized in that** it has a memory for storing the chronology of the detected events, the contents of the memory being suitable for being read, e.g. after one night.

## Patentansprüche

1. Gerät zur Bereitstellung von Pressluft für einen an Schlafstörungen, wie z'. B. Apnoe, leidenden Patienten, mit einer Zentraleinheit (U) und einer Drucksteuerung, einem gesteuerten Modul für die Bereitstellung von Druck (MPD), einer Maske (MVA) für die oberen Luftwege des Patienten, einer Leitung (CF) zur Bereitstellung von Luftdruck des Moduls (MPD) an die Maske (MVA), einen mit der Zentraleinheit (U) verbundenen Drucksensor (CDAF) der bereitgestellten Luft, und einem mit der Zentraleinheit (U) verbundenen Drucksensor (CPM) in der Maske (MVA), einer in die Zentraleinheit (U) integrierten Software, wobei die Software angeordnet ist, um
- den Luftdruck in der Maske und den der Maske bereitgestellten Luftdurchsatz zu messen,
- ausgehend von den gemessenen Variablen zu bestimmen, ob die die Schlafstörungen darstellenden Ereignisse auftreten oder nicht,
**dadurch gekennzeichnet, dass** die Software angeordnet ist, um:
- ausgehend von gemessenen Variablen zu bestimmen, ob der aktuelle Atmungszyklus des Patienten einem vorbestimmten gültigen Atmungszyklus entspricht;
- einen Auftrittsindikator (BLN) der Störungen in einen ersten Auftrittszustand (ON) der Störungen zu versetzen, wenn das Auftreten eines oder mehrerer der die Schlafstörung darstellende(n) Ereignis(se) bestimmt wird; und
- den Indikator (BLN) in einen zweiten Zustand (OFF) des Ausbleibens der Störungen versetzen zu können,
- eine erste Anzahl (CCAR) von gültigen, seit der letzten Drucksteuerung bestimmten Atmungszyklen zu zählen,
- eine dritte Anzahl (RC) von aufeinander folgenden Übergängen des Indikators (BLN) vom zweiten Zustand (OFF) in den ersten Zustand (ON) zu zählen;
- eine erste bestimmte Erhöhung des bereitgestellten Luftdrucks zu steuern (C1), wenn gleichzeitig:
* der aktuelle Atmungszyklus als gültig bestimmt wurde;
* die erste Anzahl (CCAR) größer als eine vorbestimmte erste ganze Zahl (RP) ist;
* die dritte Anzahl (RC) größer als oder gleich eine(r) dritte(n) vorbestimmte(n) ganze(n) Zahl (X) ist.

2. Gerät gemäß Anspruch 1, **dadurch gekennzeichnet, dass** die Software angeordnet ist, um
- eine zweite Zahl (CCON) von bestimmten, gültigen Atmungszyklen seit dem letzten Übergang des Indikators (BLN) in den ersten Zustand (ON) zu zählen
- die erste bestimmte Druckerhöhung von bereitgestellter Luft zu steuern, wenn darüber hinaus:
- die zweite Zahl (CCON) einer anderen oder mehreren zweiten ganzen vorbestimmten Zahlen (N) entspricht.

3. Gerät gemäß Anspruch 2, **dadurch gekennzeichnet, dass** die zweiten ganzen Zahlen (N) zwischen 1 und 300 inbegriffen sind und z. B. die drei ersten Vielfachen einer bestimmten ganzen Zahl (N₀) sind.

4. Gerät gemäß Anspruch 3, **dadurch gekennzeichnet, dass** die zweiten ganzen Zahlen (N) jeweils 2, 4 und 6 sind.

5. Gerät gemäß Anspruch 1 bis 4, **dadurch gekennzeichnet, dass** die vorbestimmte erste ganze Zahl (RP) zwischen 1 und 255 inbegriffen ist.

6. Gerät gemäß Anspruch 5, **dadurch gekennzeichnet, dass** die vorbestimmte erste ganze Zahl (RP) gleich 10 ist.

7. Gerät gemäß Anspruch 1 bis 6, **dadurch gekennzeichnet, dass** die vorbestimmte dritte ganze Zahl (X) zwischen 1 und 100 inbegriffen ist.

8. Gerät gemäß Anspruch 7, **dadurch gekennzeichnet, dass** die vorbestimmte dritte ganze Zahl (X) gleich 1 ist.

9. Gerät gemäß Anspruch 1 bis 8, **dadurch gekennzeichnet, dass** die bestimmte erste Steuerung der Druckerhöhung niedriger ist als + 10 mbar.

10. Gerät gemäß Anspruch 8, **dadurch gekennzeichnet, dass** die erste bestimmte Steuerung (C1) der Druckerhöhung deutlich gleich + 0,3 mbar ist.

11. Gerät gemäß Anspruch 1 bis 10, **dadurch gekennzeichnet, dass** die Software angeordnet ist, um die ersten und dritten Anzahlen (CCAR; RC) gezählter, gültiger Atmungszyklen und gezählter Übergänge wieder auf 0 zu setzen, nachdem die zweite gezählte Anzahl (CCON) gültiger Zyklen die größere der vorbestimmten zweiten ganzen Zahlen (N) erreicht hat und die zweite gezählte ganze Zahl (CCON) wird wieder auf 0 zu setzen, wenn der Indikator (BLN) vom zweiten Zustand (OFF) in den ersten Zustand (ON) übergeht.

12. Gerät gemäß Anspruch 1 bis 11, **dadurch gekennzeichnet, dass** der vorbestimmte gültige Atmungszyklus einem Maximum eines Atmungsdurchsatzes entspricht, der größer ist als ein vorbestimmter Durchsatzwert, wie z. B. 50 ml/Sek., einem Einatmungsvolumen, das größer ist als ein vorbestimmter Volumenwert, wie z. B. 0,05 1 und einer fehlenden Saturation beim Feststellen des Durchsatzes.

13. Gerät gemäß Anspruch 1 bis 12, **dadurch gekennzeichnet, dass** die Software angeordnet ist, um
- beim Einschalten des Geräts eine Zustandsvariable (ER) in einem dritten Zustand (NIR) ausbleibender Bearbeitung und den Indikator (BLN) im zweiten Zustand (OFF) zu initialisieren, dann sequentiell,
- ausgehend von gemessenen Variablen die Atmungszyklen als zu unterschiedlichen Kategorien gehörend zu qualifizieren, wie z. B. Zyklus mit eingeschränktem Durchsatz, intermediärer Zyklus, normaler Zyklus und nicht gültiger Zyklus, die jeweils den Gewichtungen (RSVO, REVO; RSV1; REV1, RSV2; 0, 0) entsprechen;
- die Gewichtungen der Kategorie des aktuell qualifizierten Zyklus dem ersten und zweiten Gewichtungs-Akkumulator (SV; EV) zuzuordnen;
- wenn der qualifizierte Zyklus der Kategorie des nicht gültigen Zyklus angehört, die Zustandsvariable (ER) wieder in den dritten Zustand (NIR) zu versetzen und den Indikator (BLN) in den zweiten Zustand (OFF) und einen ersten Zähler (FLC) in einem vorbestimmten Wert zu initialisieren;
- wenn der Zustand der Zustandsvariablen (ER) dem dritten Zustand (NIR) entspricht;
* wenn der Wert eines ersten Akkumulators (SV) niedriger ist als ein erster Vergleichswert, den Zähler (FLC) in seinem vorbestimmten Wert zu reinitialisieren;
* wenn der Wert des ersten Akkumulators (SV) größer als sein erster Vergleichswert ist, die Zustandsvariable (ER) in einen vierten Zustand (PR) mit einer Bearbeitungsmöglichkeiten zu versetzen und den Indikator (BLN) in den zweiten Zustand (OFF) zu versetzen;
- wenn der Zustand der Zustandsvariablen (ER) dem vierten Zustand (PR) entspricht und
* wenn der Wert des ersten Akkumulators (SV) niedriger als sein erster Vergleichswert ist, den ersten Zähler (FLC) in seinem vorbestimmten Wert zu reinitialisieren, die Zustandsvariable (ER) und den Indikator (BLN) jeweils in den dritten und vierten Zustand (NIR; OFF) zu versetzen;
* wenn der Wert des ersten Akkumulators (SV) höher ist als sein erster Vergleichswert, den ersten Zähler (FLC) seinen vorherigen Wert zuzüglich des Wertes des ersten Akkumulators (SV) annehmen zu lassen und wenn dann der Wert des ersten Zähler (FLC) größer als oder gleich eine(m) vorbestimmten / vorbestimmter oberen / oberer Anschlag (RMS) ist;
- einen zweiten Zähler (NC) in einem vorbestimmten Wert zu initialisieren;
- die Zustandsvariable (ER) in einen fünften Bearbeitungszustand (IR) übergehen zu lassen; und
- den Indikator (BLN) in den ersten Zustand (ON) übergehen zu lassen;
- wenn der Zustand der Zustandsvariablen (ER) dem fünften Bearbeitungszustand (IR) entspricht;
* wenn der Wert des zweiten Akkumulators (EV) höher ist als ein zweiter Vergleichswert, den zweiten Zähler (NC) seinen vorherigen Wert zuzüglich des Wertes des zweiten Akkumulators (EV) annehmen zu lassen und
* wenn dann der Wert des zweiten Zählers (NC) höher als der unter Anschlag (RME) oder gleich dem unteren Anschlag (RME) ist, die Zustandsvariable (ER) und den Indikator (BLN) jeweils in den dritten und zweiten Zustand (NIR; OFF) zu versetzen und den ersten und zweiten Zähler (FLC; NC) in ihren jeweiligen vorbestimmten Werten zu reinitialisieren;
* oder andernfalls den Indikator (BLN) in seinen ersten Zustand (ON) wieder zurückzuversetzen;
* wenn der Wert des zweiten Akkumulators (EV) niedriger ist als sein zweiter Vergleichswert, den zweiten Zähler (NC) in seinem jeweiligen vorbestimmten Wert zu reinitialisieren und den Indikator (BLN) in den ersten Zustand (ON) zu versetzen.

14. Gerät gemäß Anspruch 13, **dadurch gekennzeichnet, dass** die den Kategorien normaler Zyklus, intermediärer Zyklus, Zyklus mit eingeschränktem Durchsatz und nicht gültiger Zyklus entsprechenden Gewichtungen (RSV2, REV2, RSV1, REV1; RSV0; REV0; 0, 0) beim ersten Akkumulator (SV) jeweils deutlich gleich - 1; 1; 5 und 0 sind und beim zweiten Akkumulator (EV) jeweils deutlich gleich 1; -1; - 1 und 0 sind; die ersten und zweiten Vergleichswerte und die vorbestimmten Initialisierungswerte des ersten und zweiten Zählers (FLC; NC) jeweils deutlich gleich 0 und der hohe und der niedrige Anschlag (RMS; RME) jeweils deutlich gleich 10 und 2 sind.

15. Gerät gemäß Anspruch 1 bis 14, **dadurch gekennzeichnet, dass**
- der vorbestimmte gültige Atmungszyklus einem Maximum eines Einatmungsdurchsatzes oberhalb eines vorbestimmten Durchsatzwertes entspricht, wie z. B. 50 ml/Sek. einem Einatmungsvolumen oberhalb eines vorbestimmten Volumenwertes, wie z. B. 0,05 1, einer fehlenden Saturation bei der Feststellung des Durchsatzes, einer in einem vorbestimmten Intervall inbegriffenen gemessenen Einatmungszeit, wie z. B. 0,5 Sek. bis 6 Sek. und einer in einem anderen vorbestimmten Intervall inbegriffenen gemessenen Dauer von Einatmungszyklen, wie z. B. 1,5 Sek. bis 20 Sek.;
wenn der gemessene Atmungszyklus als gültig bestimmt wird, dann
- berechnet die Software eine vorbestimmte Merkmale im Verhältnis zur Einatmungskurve des gemessenen Einatmungszyklus respektierende äquivalente sinusähnliche Kurve;
- berechnet die Software ein im Verhältnis zum durch die Einatmungskurve auf dem begrenzten Bereich durch die äquivalente sinusähnliche Kurve begrenzten Bereich proportionales Flächenkriterium CS; wobei jede in ein und demselben, in der Einatmungsphase des gemessenen Atmungszyklus inbegriffenen Zeitintervall berücksichtigt wird;
- berechnet die Software ein Korrelationskriterium (CC) zwischen der Einatmungskurve des gemessenen Einatmungszyklus und der äquivalenten sinusähnlichen Kurve;
- wenn das berechnete Korrelationskriterium (CC) größer als oder gleich eine(r) erste(n) normale(n) vorbestimmte(n) Grenze (LN) ist und wenn das berechnete Flächenkriterium (CS) größer als eine zweite vorbestimmte Flächengrenze (LS) ist, qualifiziert die Software den gemessenen Atmungszyklus als normal und andernfalls qualifiziert die Software den Zyklus als mit eingeschränktem Durchsatz:
- wenn der gemessene Atmungszyklus als Zyklus mit eingeschränktem Durchsatz qualifiziert wurde;
* wenn das gemessene Flächenkriterium (CS) eine dritte vorbestimmte Expertengrenze (LE) übersteigt, qualifiziert die Software den gemessenen Atmungszyklus als normal;
* oder andernfalls,
* wenn das berechnete Flächenkriterium (CS) eine vierte vorbestimmte Durchsatzgrenze (LD) übersteigt, qualifiziert die Software den gemessenen Atmungszyklus wieder als intermediär,
* und im entgegen gesetzten Fall qualifiziert die Software den Zyklus als Zyklus mit eingeschränktem Durchsatz,
wobei die zweite Flächengrenze (LS) die vierte Durchsatzgrenze (LD) und die dritte Expertengrenze (LE) in aufsteigender Ordnung vorbestimmt sind.

16. Gerät gemäß Anspruch 15, **dadurch gekennzeichnet, dass**
- die vorbestimmten Merkmale der äquivalenten sinusähnlichen Kurve eine der gemessenen Einatmungszeit deutliche gleiche halbe Periode und eine deutlich denselben Ursprung aufweisende absteigende Kurve umfassen wie die der Einatmungskurve, wenn sie deutlich ein Drittel ihrer maximalen Amplitude erreicht;
- das berechnete Oberflächenkriterium (CS) deutlich gleich dem Hundertfachen des Verhältnisses der jeweils zwischen einem Viertel und einem Dreiviertel der Dauer der Einatmungsphase des gemessenen Ausatmungszyklus berücksichtigten Grundflächen ist;
- das berechnete Korrelationskriterium (CC) deutlich gleich dem Maximum des Hundertfachen der Korrelationskoeffizienten zwischen der Einatmungskurve und der äquivalenten sinusähnlichen Kurve ist, die jeweils in der zweiten Hälfte der Einatmungsphase und deren Gesamtheit berücksichtigt werden;
- wobei die ersten, zweiten, vierten und dritten Grenzen (LN; LS; LD; LE) jeweils zwischen 45 und 100; 0 und 100; 0 und 100; 0 und 100 inbegriffen sind und z. B. deutlich jeweils gleich 87; 40; 60 und 90 sind.

17. Gerät gemäß Anspruch 1 bis 16, **dadurch gekennzeichnet, dass** die Software angeordnet ist, um
- bei jedem Ende des gemessenen Ausatmungszyklus die durchschnittliche Amplitude (AM) auf einer vierten vorbestimmten Zahl (Y4) von vorherigen Ausatmungszyklen zu berechnen,
- wenn die gemessene Amplitude des letzten Ausatmungszyklus niedriger ist als die berechnete, durchschnittliche Amplitude (AM), multipliziert mit einem ersten vorbestimmten Hypopnoe-Faktor (FHO), wird zu einem Zeitzähler (CTHO) in Hypopnoe die Dauer (TC) des letzten gemessenen Ausatmungszyklus hinzugefügt,
* wenn der aktuelle Wert des Zeitzählers in Hypopnoe (CTHO) größer als oder gleich eine(r) Mindest-Hypopnoe-Zeit (TMHO) ist, eine fünfte vorbestimmte Druckerhöhung (C5) zu steuern,
* nach dem Ende einer fünften vorbestimmten Anzahl (Y5) von Ausatmungszyklen gemäß der fünften Steuerung (C5) der Druckerhöhung, eine sechste vorbestimmte Druckerhöhung (C6) zu steuern,
* Nach dem Ende einer sechsten vorbestimmten Anzahl von Ausatmungszyklen (Y6), die größer ist als die fünfte Anzahl (Y5) gemäß der fünften Steuerung (C5) der Druckerhöhung, eine siebte Druckerhöhung (C7) zu steuern,
- wobei der Zeitzähler in Hypopnoe (CTHO) beim Einschalten des Geräts bei 0 initialisiert wird.

18. Gerät gemäß Anspruch 17, **dadurch gekennzeichnet, dass**:
- die vierte vorbestimmte Anzahl (Y4) von Berechnungs-Ausatmungszyklen durchschnittlicher Amplitude deutlich gleich 8 ist,
der erste vorbestimmte Hypopnoe-Faktor (FHO) zwischen 1 und 100 % inbegriffen ist und z. B. deutlich gleich 40 % ist,
die Mindest-Hypopnoe-Zeit (TMHO) zwischen 1 Sek. und 25 Sek. inbegriffen ist und z. B. deutlich gleich 10 Sek. ist,
die vorbestimmten fünften und sechsten Anzahlen (Y5; Y6) von Ausatmungszyklen jeweils deutlich gleich 2 und 4 sind,
die vorbestimmte fünfte Druckerhöhung (C5) zwischen 0,1 mbar und 10 mbar inbegriffen ist und z. B. deutlich gleich + 1 mbar ist,
wobei die sechsten und siebten vorbestimmten Druckerhöhungen (C6; C7) jeweils niedriger sind als die fünfte Steuerung (C5) und z. B. jeweils deutlich gleich der Hälfte der fünften Druckerhöhung (C5) sind.

19. Gerät gemäß Anspruch 18, **dadurch gekennzeichnet, dass** die Software angeordnet ist, um
- wenn die gemessene Amplitude des letzten Ausatmungszyklus größer als oder gleich / die der durchschnittliche(n) berechnete(n) Amplitude (AM) multipliziert mit dem ersten Hypopnoe-Faktor (FHO) ist, dann die durchschnittliche Zeit (TCM) der Ausatmungszyklen aus einer siebten vorbestimmten Anzahl (Y7) von vorherigen Zyklen zu berechnen:
* wenn die gemessene Dauer (TC) des letzten Zyklus länger ist als eine vorbestimmte achte Anzahl (Y8), multipliziert mit der Zeit des durchschnittlichen berechneten Ausatmungszyklus (TCM), zum Zeitzähler in Hypopnoe (CTHO) die gemessene Dauer (TC) des letzten Zyklus hinzuzufügen, multipliziert mit einem zweiten Hypopnoe-faktor (F2),
* wenn die gemessene Amplitude des letzten gemessenen Ausatmungszyklus größer ist als ein dritter Hyperventilationsfaktor (F3), der größer ist als ein erster Hypopnoefaktor (FHO), multipliziert mit der berechneten durchschnittlichen Amplitude (AM), den letzten hyperventilierten Zyklus zu qualifizieren, einen Zähler hyperventilierter Zyklen (CCH) um eine Einheit zu inkrementieren, einen Zähler (CCN) von Zyklen mit normaler Ventilation wieder auf 0 zu setzen und
* wenn der Wert des Zählers (CCH) hyperventilierter Zyklen größer als oder gleich eine(r) vorbestimmte(n) neunte(n) Anzahl (Y9) ist,
* wenn die Dauer des letzten Zyklus (TC) länger als oder gleich der achten Anzahl (Y8) multipliziert mit der berechneten durchschnittlichen Zykluszeit (TCM) ist, zum Zeitzähler (CTHO) in Hypopnoe den zweiten Faktor (F2) multipliziert mit der Dauer des zweiten Ausatmungszyklus (TC) hinzuzufügen
* und andernfalls den Zeitzähler (CTHO) in Hypopnoe wieder auf 0 zu setzen;
anschließend einen Zähler (CCHO von) hypoventilierten Zyklen wieder auf 0 setzen und die durchschnittliche Amplitude (AM) von Atmungszyklen aus der vorbestimmten Anzahl (Y4) von vorherigen Atmungszyklen zu berechnen,
* wenn die gemessene Amplitude des letzten Atmungszyklus kleiner als oder gleich dem drittem Faktor (F3) multipliziert mit der durchschnittlichen berechneten Amplitude (AM), den letzten Zyklus mit normaler Ventilation zu qualifizieren, den Zähler (CCH) von hyperventilierten Zyklen wieder auf 0 setzen und den Zähler (CCN) von Zyklen mit normaler Ventilation um eine Einheit zu inkrementieren, und
* wenn der Wert des Zählers (CCN) von Zyklen mit normaler Ventilation größer als oder gleich eine(r) vorbestimmte(n) zehnte(n) Anzahl (Y10) ist,
* wenn die Dauer des letzten Zyklus (TC) größer als oder gleich die / der achte(n) Anzahl (Y8) multipliziert mit der berechneten durchschnittlichen Zykluszeit (TCM) ist, dem Zeitzähler (CTHO) in Hypopnoe den zweiten Faktor (F2) multipliziert mit der Dauer des letzten Zyklus (TC) zuzuordnen und den Zähler (CCN) von Zyklen mit normaler Ventilation wieder auf 0 zu setzen,
* und andernfalls den Zeitzähler (CTHO) in Hypopnoe wieder auf 0 zu setzen, dann den Zähler (CCHO) von hypoventilierten Zyklen wieder auf 0 zu setzen und die durchschnittliche Amplitude des Atmungszyklus aus der vorbestimmten Anzahl (Y4) von Atmungszyklen zu berechnen.

20. Gerät gemäß Anspruch 19, **dadurch gekennzeichnet, dass** der zweite Faktor (F2) deutlich gleich 5/8 ist, der dritte Hyperventilationsfaktor (F3) zwischen 100 % und 200 % inbegriffen ist und z. B. deutlich gleich 140 % ist,
die siebten, achten, neunten und zehnten vorbestimmten Anzahlen (Y7; Y8; Y9; Y10) jeweils deutlich gleich 3; 2; 2 und 2 sind.

21. Gerät gemäß Anspruch 1 bis 20, **dadurch gekennzeichnet, dass** die Software angeordnet ist, um
- wenn der gemessene Druck (P) unter einem vorbestimmten hohen Druckwert (PH) liegt, einen Zeitzähler hohen Drucks (TPH) wieder auf 0 zu setzen;
- wenn der Wert des Zeitzählers bei hohem Druck (TPH) höher als eine maximale Zeit bei hohem Druck (TMPH) ist und
* wenn der maximale Wert des eingestellten Drucks (Pmaxi) unter einem vorbestimmten Sicherheitsdruckwert (PSEC) liegt, den Druck (P) dieses maximalen Wertes des eingestellten Drucks (Pmax) zu steuern;
* wenn der minimale Wert des eingestellten Drucks (Pmini) höher als ein vorbestimmter Sicherheitsdruckwert (PSEC) ist, den Druck (P) bei diesem minimalen Wert des eingestellten Drucks (Pmini) zu steuern;
* wenn die beiden vorherigen Bedingungen nicht erfüllt sind, den Druck (P) bei dem Sicherheitsdruckwert (PSEC) zu steuern
dann
den Zeitzähler bei hohen Druck (TPH) wieder auf 0 zu setzen.

22. Gerät gemäß Anspruch 21, **dadurch gekennzeichnet, dass** der Wert hohen Drucks (PH) zwischen 10 mbar und 25 mbar inbegriffen ist und z. B. deutlich gleich 17 mbar ist,
die maximale Zeit hohen Drucks (TMPH) zwischen 1 und 100 Minuten inbegriffen ist und z. B. deutlich gleich 10 Minuten oder 30 Minuten ist,
der Sicherheitsdruckwert (PSEC) deutlich gleich 8 mbar ist.

23. Gerät gemäß Anspruch 1 bis 22, **dadurch gekennzeichnet, dass** die Software angeordnet ist, um einen Luftaustritt zu messen, der deutlich gleich dem durchschnittlichen Durchsatz während der Atmung des Patienten ist,
- wenn der gemessene Luftaustritt größer ist als ein vorbestimmtes Austrittsniveau (NFM), die Druckerhöhungssteuerungen ungültig zu machen.

24. Gerät gemäß Anspruch 23, **dadurch gekennzeichnet, dass** das vorbestimmte Austrittsniveau (NFM) deutlich gleich einem Austrittskoeffizienten (A) multipliziert mit einem gefilterten Luftdruck in der Maske ist, zuzüglich eines zusätzlichen Austrittskoeffizienten (B), wobei der Austrittskoeffizient (A) zwischen 0 und 10 l/Min mbar inbegriffen ist und z. B. deutlich gleich 2,5 l/Min. mbar ist und
der zusätzliche Austrittskoeffizient (B) zwischen 0 und 100 1/Min. inbegriffen ist und z. B. deutlich gleich 50 1/Min. ist.

25. Gerät gemäß Anspruch 1 bis 24, **dadurch gekennzeichnet, dass** die Software angeordnet ist,
- um festzustellen, ob die gemessene Druckkurve Schwankungen aufweist, wie z. B. in einem Frequenzbereich (P1) inbegriffenen akustische Vibrationen,
die Zeit (RF1) des Vorhandenseins von zwischen zwei aufeinander folgenden fehlenden festgestellten Schwankungen und der Zeit (RF0) festgestellter fehlender Schwankungen zwischen zwei festgestellten aufeinander folgenden vorhandenen Schwankungen festgestellten Schwankungen zu messen;
- ob die Summe der gemessenen Zeiten der festgestellten fehlenden und vorhandenen Schwankungen (RF0; RF1) in einem vorgeschriebenen zeitlichen Bereich (BP; BSP) inbegriffen ist;
- ob die gemessene Zeit der vorhandenen Schwankungen (RF1) länger als oder gleich einer MindestSchwankungszeit (TMRH) ist und
- ob der Wert eines Zählers (CTAR) der seit dem vorletzten Mal, als die vorherigen zeitlichen Bedingungen erfüllt wurden, abgelaufenen Zeit länger ist als eine vorgeschriebene Wartezeit (TAR), eine achte vorbestimmte Druckerhöhung zu steuern (C8) und den Zähler der abgelaufenen Zeit (CTAR) wieder auf 0 zu setzen.

26. Gerät gemäß Anspruch 25, **dadurch gekennzeichnet, dass** die Software angeordnet ist, um
- wenn die Summe der gemessenen Zeiten der festgestellten vorhandenen und fehlenden Schwankungen (RF0; RF1) den vorgeschriebenen zeitlichen Bereich (BIP; BSP) über- oder unterschreitet oder wenn die gemessene Zeit der festgestellten vorhandenen Schwankungen (RF1) unterhalb einer Mindestschwankungszeit (TMRH) liegt,
* die gemessene Zeit (RF0) festgestellter fehlender Schwankungen durch die Summe der gemessenen Zeiten festgestellter fehlender und vorhandener Schwankungen (RF0; RF1) zu ersetzen, dann
* die gemessene Zeit festgestellter vorhandener Schwankungen (RF1) wieder auf 0 zu setzen und
- wenn die Summe der gemessenen Zeiten festgestellter fehlender und vorhandener Schwankungen (RF0; RF1) den vorbestimmten zeitlichen Bereich (BIP; BSP) oder eine vorbestimmte maximale Zeit (TCMax) über- oder unterschreitet, jede der gemessenen Zeiten festgestellter fehlender und vorhandener Schwankungen (RF0; RF1) wieder auf 0 zu setzen; und andernfalls
- jede der gemessenen Zeiten festgestellter fehlender und vorhandener Schwankungen (RF0; RF1) wieder auf 0 zu setzen.

27. Gerät gemäß Anspruch 25 und 26, **dadurch gekennzeichnet, dass**:
die vorbestimmte maximale Zeit deutlich gleich dem Doppelten der Zeit (TCM) des durchschnittlichen Ausatmungszyklus der letzten drei gemessenen Zyklen ist
- der vorgeschriebene zeitliche Bereich (BIP; BSP) deutlich zwischen 10 % und 120 % der berechneten durchschnittlichen Zykluszeit (TCM) inbegriffen ist;
- die Mindest-Schwankungs-Zeit (TMRH) deutlich gleich 7 % der berechneten durchschnittlichen Zykluszeit (TCM) ist;
- die vorgeschriebene Wartezeit (TAR) zwischen 1 und 30 Minuten inbegriffen und ist z. B. deutlich gleich 1 Minute ist;
- die achte Steuerung (C8) der Druckerhöhung zwischen 0,1 mbar und 120 mbar inbegriffen und z. B. deutlich gleich 1 mbar ist;
- der Frequenzbereich (P1) zur Feststellung von Schwankungen deutlich zwischen 30 und 300 Hz inbegriffen ist.

28. Gerät gemäß Anspruch 1 bis 27, **dadurch gekennzeichnet, dass** die Software angeordnet ist, um die Chronologie der festgestellten Ereignisse zu speichern und man z. B. nach einer Nacht die gespeicherte Chronologie abliest.

29. Gerät gemäß Anspruch 1 bis 28, **dadurch gekennzeichnet, dass** es einen Speicher der Chronologie der festgestellten Ereignisse umfasst, dessen Inhalt geeignet ist, z. B. nach einer Nacht abgelesen zu werden.
